# EUROPEAN PATENT SPECIFICATION

(11) **EP 1 773 368 B1**
(45) Date of publication and mention of the grant of the patent: **04.05.2016**
(21) Application number: 05789138.4
(22) Date of filing: 28.06.2005
(51) Int. Cl.: A61K 38/00

(54) **A METHOD OF PRE-SENSITIZING CANCER PRIOR TO TREAMENT WITH RADIATION AND/OR CHEMOTHERAPY AND A NOVEL CYTOKINE MIXTURE**
VERFAHREN ZUR PRÄSENSIBILISIERUNG VON KREBS VOR EINER STRAHLENBEHANDLUNG UND/ODER CHEMOTHERAPIE UND NEUE ZYTOKINMISCHUNG
PROCEDE DE PRESENSIBILISATION DE CANCERS AVANT UNE RADIOTHERAPIE ET/OU UNE CHIMIOTHERAPIE, ET NOUVEAU MELANGE DE CYTOKINES

(30) Priority: 29.06.2004 US 878563
(43) Date of publication of application: 18.04.2007
(73) Proprietor: Cel-Sci Corporation, Vienna, VA 22182 (US)
(72) Inventor: TALOR, Eyal, Baltimore, MD 21209 (US)
(74) Representative: Potter Clarkson LLP
(86) International application number: PCT/US2005/022678
(87) International publication number: WO 2006/004633

(56) References cited:
- WO-A2-2005/007086
- FEINMESSER ET AL: 'Report of a Clinical Trial in 12 Patients With Head and Neck Cancer Treated Intratumorally and Peritumorally With Multikine.' ARCH OTOLARYNGOL. vol. 129, August 2003, pages 874 - 881, XP008059890
- TIMAR ET AL: 'The Effect of Leukocyte Interleukin Injection (Multikine)Treatment on the Peritumoral and Intratumoral Subpopulation of Mononuclear Cells and on Tumor Epithelia: A Possible New Approach to Augmenting Sensitivity to Radiation Therapy and Chemotherapy in Or al Cancer.' LARYNGOSCOPE. vol. 113, December 2003, pages 2206 - 2217, XP008048121
- TÍMÁR JÓZSEF ET AL: "Neoadjuvant immunotherapy of oral squamous cell carcinoma modulates intratumoral CD4/CD8 ratio and tumor microenvironment: a multicenter phase II clinical trial", JOURNAL OF CLINICAL ONCOLOGY, AMERICAN SOCIETY OF CLINICAL ONCOLOGY, US, vol. 23, no. 15, 20 May 2005 (2005-05-20), pages 3421-3432, XP002552350, ISSN: 0732-183X, DOI: DOI:10.1200/JCO.2005.06.005
- G. TAYLOR: 'Immunotherapy with Leucocyte Interleukin, Injection for Human Papilloma Virus (HPV) Induced Cervical Dysplasia in HIV patients' POSTER PRESENTATION 33RD ANNUMAL MEETING OF THE SOCIETY OF GYNECOLOGIC ONCOLOGISTS MIAMI FLORIDA USA 16 March 2002, XP055157030

## Description

### INTRODUCTION

This invention relates to a breakthrough use of a novel cytokine mixture for pre-sensitizing oral squamous cell carcinoma tumours (OSCCT) prior to a therapeutic treatment such as chemotherapy, radiation therapy or immunotherapy. The cytokine mixture is a serum-free and mitogen-free mixture comprised of specific ratios of cytokines such as IL-1β, TNF-α, IFN-γ and GM-CSF to Interleukin 2 QDL-2), which is effective in inducing cancerous cells to enter a proliferative cell cycle phase thereby increasing their vulnerability to chemotherapy, radiation therapy and immuno-therapy. One such novel cytokine mixture is Leukocyte Interleukin Injection (LI) or Multikine® which can be used alone or in combination with other drugs for the treatment of cancer thereby increasing the success of cancer treatment and the disease free survival of cancer patients.

### BACKGROUND OF THE INVENTION

Current treatments of cancer and in particular solid tumors, comprise mainly of surgical intervention followed by radiation therapy and/or chemotherapy. Dunne-Daly CF, "Principles of radiotherapy and radiobiology", Semin Oncol Nurs. 1999 Nov;15(4):250-9; Hensley ML et al., "American Society of Clinical Oncology clinical practice guidelines for the use of chemotherapy and radiotherapy protectants.", J Clin Oncol. 1999 Oct; 17(10): 3333-55. In conjunction with such therapies, toxic chemotherapeutic agents such as Gemcidabin, Vinblastine, Cisplatin, Fluorouracil, Gleevec, Methotrexate, which are unable to differentiate between normal and cancerous cells are used. While effective, these and other toxic chemotherapeutic agents have done little to increase overall patient survival. Moreover, current treatments in general also failed to improve 5-year survival rate of cancer patients despite synergistic combination of chemotherapies and radiotherapies. Even anti-epidermal growth factor receptor agents, anti-angiogenic drugs and immuno and immuno-adjuvant therapy using drugs such as Rituximab, Erbitux and Herceptin have failed to significantly increase the 5-year survival rate of cancer patients. Furthermore, complete remission or disease free survival of cancer patient irrespective of cancer type have not been improved upon by any of the aforementioned therapies or synergistic combinations thereof.

One treatment modality investigated to improve disease-free survival rates or lead to complete remission is manipulation of cell-division cycle of cancerous cells. In particular, cycling tumor cells are generally more vulnerable to radio- and chemotherapies than non-cycling tumor cells because complex biochemical and biomolecular processes such as enzyme-dependent DNA replication, enzyme-dependent phosphorylation, signal cascades, association and dissociation of transcriptional activating molecular complexes, and formation and dissociation of macromolecular assemblies of cytostructural elements are required during cell cycling. By inducing tumor cells into a cell cycle phase, anti-metabolic agents that inhibit any of the complex biochemical processes such as ribonucleotide reductase (RNR) inhibitors, dihydrofolate reductase inhibitors or DNA polymerase inhibitors can be used to stop cell cycling and thereby prevent tumor proliferation.

However, known methods taking advantage of cell cycling are limited to synchronizing cell cycle arrest with sequential applications of a chemotherapeutic agent. For example, one known method arrests malignant cells within a S phase of the cell cycle with pyrimidine analogs followed by exposure to high concentrations of anti-metabolites. B. Bhutan et al., Cancer Res. 33:888-894 (1973). Few or no cells in the population can proceed beyond this point of detention after application of the antimetabolite. W Vogel et al., Hum. Genet. 45:193-8 (1978).

Other efforts include methods of manipulating the cell-division cycle by altering the cell cycle distribution within the cell population. These protocols stimulate malignant cells from a dormant phase into a cell cycling phase thereby increasing their vulnerability to anti-metabolic drugs acting during the vulnerable DNA replication phase. H H Euler et al., Ann. Med. Interne. (Paris) 145:296-302 (1994); B C Lampkin et al., J. Clin. Invest. 50:2204-14 (1971); Alama et al., Anticancer Res. 10:853-8 (1990). Conversely, other known methods prohibit normal cells from entering S phase thereby protecting normal cells from chemotactic drugs.

Still another known method of synchronizing cell cycle phase with chemotherapeutics is a so-called pulse dose chemotherapy described by R E Moran et al., Cancer Treat. Rep. 64:81-6 (1980). In this approach, leukemic tumor cells in mice were detained in a S phase of the cell cycle with an infusion of hydroxyurea. After the infusion, the cells were "released" to continue cell cycling wherein a "pulse" of a second agent (Ara-C) was given to the mice. The intent was to maximize impact of the second agent as the cycling cells were moving through the vulnerable cell cycle S-phase. However, the results indicated that while mice treated with Ara-C just after the hydroxyurea infusion showed improved survival, mice treated with Ara-C at later times after the hydroxyurea infusion did not show improved survival. Clearly, simply synchronizing cell cycling with a second agent acting non-simultaneously did not improve the action of the two agents.

Nevertheless, known methods taking advantage of cell cycle continue to seek an optimal but passive synergy between dosage, pharmacokinetics, sequence and scheduling.

It might be expected that confining a cell population to a vulnerable cell cycle phase where cells are specifically vulnerable to damage might shift the dynamics of cell killing toward greater efficiency with a greater reduction in side-effects by diminishing exposure to toxic drugs. However, actual experiments taking advantage of cell cycle arrest or static synchronization have been disappointing because known methods are unable to actually induce cells into a cell cycle. Rather, all the known methods simply time the synergistic combination of cell cycle arrest or static synchronization with the target cell population. Moreover, agents such as pyrimidine and hydroxyurea used to effect the cell cycle can cause damage to normal cells.

Another approach would, of course, be to induce the cells to enter into a cell cycle phase as opposed to arresting the cell cycle or synchronizing the cell cycle. However, as would be otherwise predicted from the art, inducing cells into cell cycling increases the risk of a rapidly growing and recurring tumor. But the continued failure of known compositions to improve disease-free survival rates or lead to complete remission suggests a need for inducing malignant cells into cell cycling in a manner that does not proliferate the tumor but increases the susceptibility of the residual tumor to follow-on treatment with radiation and/or chemotherapy.

Therefore, there is a need for methods for inducing tumor cells into a cell cycle selected from the group of (different phases of the cell cycle) G₁, S, G₂ and M where the new methods can be synergistically applied with chemotherapy, immuno-therapy and radiation therapy. There is also a need for pre-sensitizing cancer tumors in general along with the need for a new serum-free and mitogen-free cytokine mixture comprised of specific ratios of IL-I β to IL-2, TNF-α to IL-2, IFN-γ to IL-2 and GM- CSF to IL-2 that unexpectedly demonstrates far better efficacy over known compositions in inducing a tumor cells to enter a cell cycle phase or for pre-sensitizing a cancer.

### SUMMARY OF THE INVENTION

The present invention is based, in part, on pre-sensitizing OSCCT using and a new serum-free and mitogen-free cytokine mixture having specific ratios of IL-I β to IL-2, TNF-α to IL-2, IFN-γ to IL-2 and GM-CSF to IL-2. Also described herein is the development of compositions useful as a pharmaceutical or as an adjuvant to be used in conjunction with therapeutic cancer treatments such as chemotherapy, immuno-therapy and radiation therapy.

Also disclosed herein is a method of improving conventional chemotherapy or radiotherapy of neoplasms or diseases of the immune system with a serum-free and mitogen-free cytokine mixture is disclosed. The methods provide for a pre-sensitizing step for the treatment of cancer in conjunction with radiotherapies or other physical modalities of cell killing. A method for inducing tumor cells into a vulnerable cell cycle phase selected from the group of (different phases of the cell cycle) G₁, S, G₂ and M is also contemplated. The present disclosure is not limited to any one particular type of cancer and can include any type of cancer. Specific applications include administering a serum-free and mitogen-free cytokine mixture peritumorally three times a week over a two week period in a range from about 20 IU to 1600 IU or specifically at 400 IU or at 800 IU or still further at five times a week in a range from about 20 IU to 1600 IU or at 400 IU or at 800 IU, wherein IU represent International Units for Interleukin-2 given in World Health Organization 1^{st} International Standard for Human IL-2, 86/504.

Also disclosed herein are serum-free and mitogen-free cytokine preparation such as Leukocyte Interleukin Injection (LI) or Multikine® in novel and non-obvious concentrations. The cytokine preparation may further be part of a pharmaceutical composition. In the present invention, the new serum-free and mitogen-free cytokine preparation has specific ratios of cytokine to interleukin 2 (IL- 2) as follows: IL-1β to IL-2 at a ratio range of 0.4 - 1.5, and preferably at 0.7+/- 0.1 (IL-1β/IL-2), TNF-α to IL-2 at a ratio range of 3.2 - 11.3, and preferably at 9.5+/- 1.8 (TNF-α/IL-2), IFN-γ to IL-2 at a ratio range of 1.5 - 10.9, and preferably at 6.0+/- 1.1 (IFN-γ/IL-2), and GM-CSF to IL-2 at a ratio range of 2.2 - 4.8, and preferably at 4.0+/- 0.5 (GM-CSF/IL-2).

In the present invention, the serum-free and mitogen-free cytokine preparation or pharmaceutical composition has further different cytokines and other small biologically active molecules in Leukocyte Interleukin Injection (LI) or Multikine® wherein the ratio of each of the small biologically active molecules to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/- 10.6, IL-1α to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16 - 2.84, preferably at 1.84+/-0.68, G-CSF to IL-2 in a ratio range of 2.16 - 3.78, preferably at 2.97+/- 0.81, TNF-β to IL-2 in a ratio range of 1.17 - 2.43, preferably at 1.8+/- 0.63, MIP-1α to IL-2 in a ratio range of 15.7 - 37.16, preferably at 22.7+/- 7.0, MIP-1β to IL-2 in a ratio range of 17.1 - 28.5, preferably at 22.8+/- 5.7, a RANTES to IL-2 in a ratio range of 2.3 - 2.7, preferably at 2.5+/- 0.13, a EGF to IL-2 in a ratio range of 0.267 - 0.283, preferably at 0.275+/- 0.008, PGE₂ to IL-2 in a ratio range of 3.63 - 5.42, preferably at 4.5+/- 0.87 and TxB₂ to IL-2 in a ratio range of 23.47 - 25.13, preferably at 24.3+/- 0.83.

Those of skill in the art who routinely treat patients with cancer are aware that there are many different regimens for the treatment of such cancers and the application of those regimens to particular patients will depend on the consideration of a variety of factors, e.g., the stage of the cancer, the extent of the spread of the cancer cells, e.g. have they metastasized, and the physical attributes of the patient. Those of skill in the art routinely adjust the parameters of a particular treatment, e.g., dose, duration, administration route and administered form, for particular patients and those parameters are adjusted without undue experimentation by one of ordinary skill in the art. The accompanying drawings and tables, which constitute a part of the disclosure, illustrate and, together with the description, explain the principle of the invention. One of ordinary skill in the art will appreciate that other aspects of this invention will become apparent upon reference to the attached figures and the following detailed description.

### BRIEF DESCRIPTION OF THE DRAWINGS

The patent or application contains at least one drawing executed in color. Copies of this application or patent application publication with color drawings(s) will be provided by the Office upon request and payment of the necessary fee.

The invention will now be explained in greater detail by the following description and specific embodiments and with the aid of the accompanying drawings.

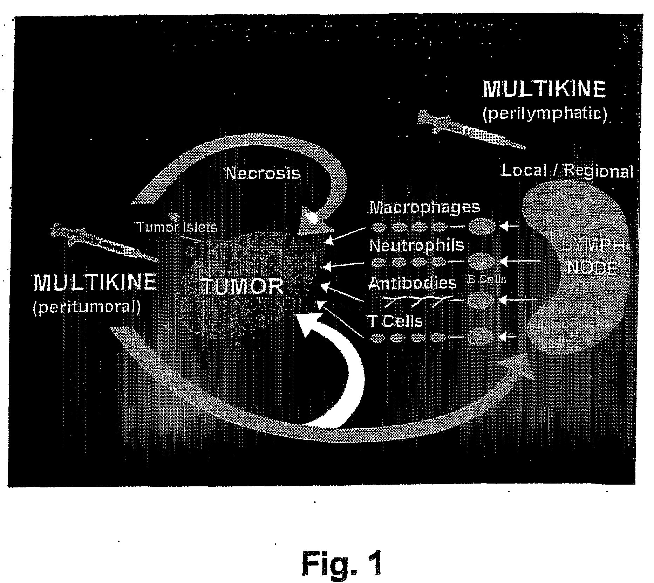

Fig. 1 represents the mode of action of Leukocyte Interleukin Injection (LI) or Multikine®.

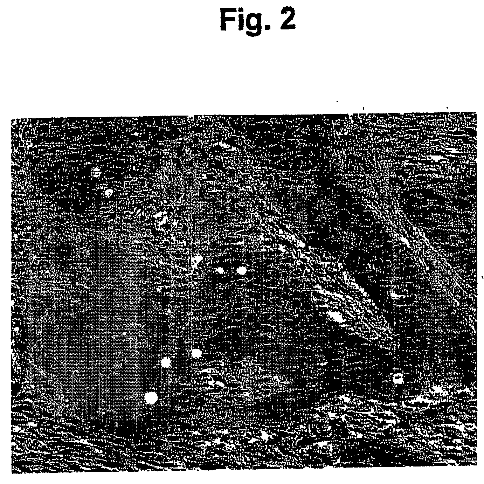

Fig. 2 represents the effect of Leukocyte Interleukin Injection (LI) treatment on the percentage of tumor cycling cells in patients having oral squamous cell carcinoma (OSCC) of the head and neck with respect to the immuno-histochemical appearance of Ki-67 - positive cells in OSCC in an LI-treated group. Data are presented as mean values ± SEM (n=25, control, n=11, LI-treated group) (**P* < 0.05) (0 = control, untreated group).

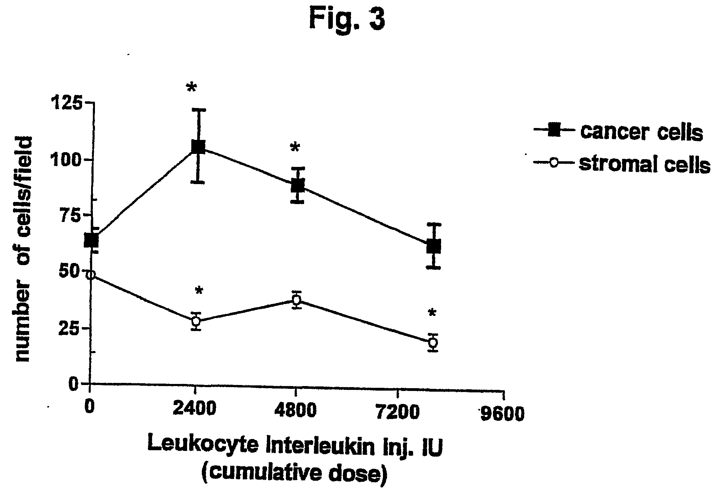

Fig. 3 represents the effect of increasing dose of Leukocyte Interleukin Injection (LI) treatment on the lymphoid cells detected by the CD45 marker with respect to the morphometry of stromal lymphoid cell density in oral squamous cell carcinoma (OSCC) at the tumor surface (zone 1.0); tumor center (zone 2.0); and tumor surface interface (zone 3.0). Data are presented as mean values ± SEM (n=27, control group, n=11, LI-treated group) (**P* < 0.05).

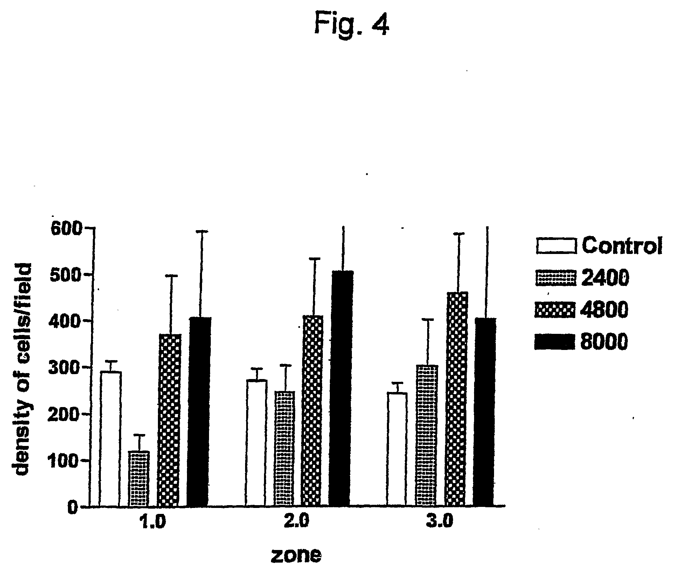

Fig. 4 represents the effect of increasing dose of Leukocyte Interleukin Injection (LI) treatment on the percentage of cycling cells in oral squamous cell carcinoma (OSCC) with respect to morphometry. Ki-67 - positive cells have been counted both in the stromal compartment and in the tumor epithelial nests. Data are presented as mean values ± SEM (n=25, control, n=11, LI-treated group) (**P* < 0.05) (0 = control, untreated group).

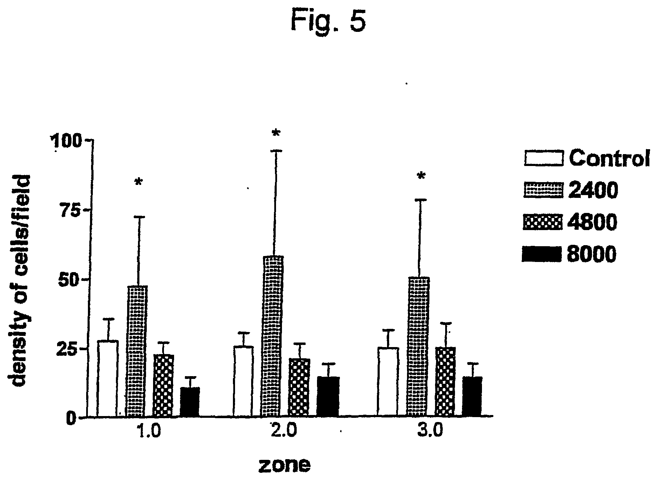

Fig. 5 represents the effect of increasing dose of Leukocyte Interleukin Injection (LI) treatment on the lymphoid cells detected by the CD45 marker with respect to the morphometry of intraepithelial lymphoid cells in OSCC. Zone 1.0 = tumor surface; Zone 2.0 = tumor center; Zone 3.0 = tumor-stroma interface. Data are presented as mean values ± SEM (n=27, control group, n=11, LI-treated group) (**P* < 0.05).

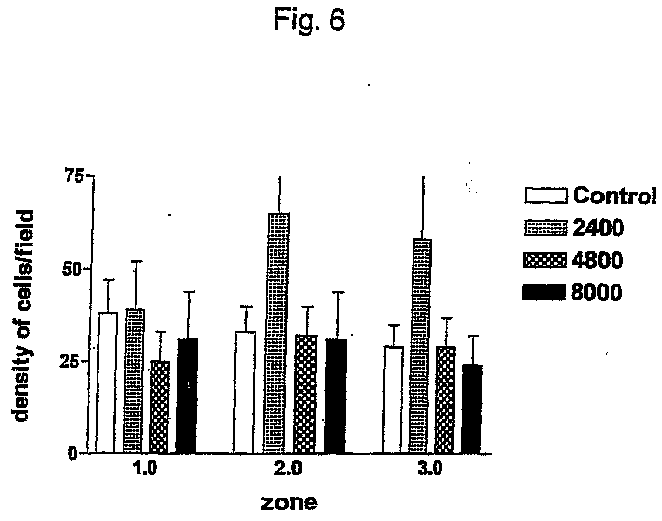

Fig. 6 represents the effect of increasing dose of Leukocyte Interleukin Injection (LI) treatment on the density of interleukin-2, receptor-positive (CD-25) lymphoid cells in oral squamous cell carcinoma (OSCC) (morphometry) on the stromal density. Zone 1.0 = tumor surface; Zone 2.0 = tumor center; Zone 3.0 = tumor-stroma interface. Data are presented as mean values ± SEM (n=27, control group; n=11, LI-treated group) (**P* < 0.05).

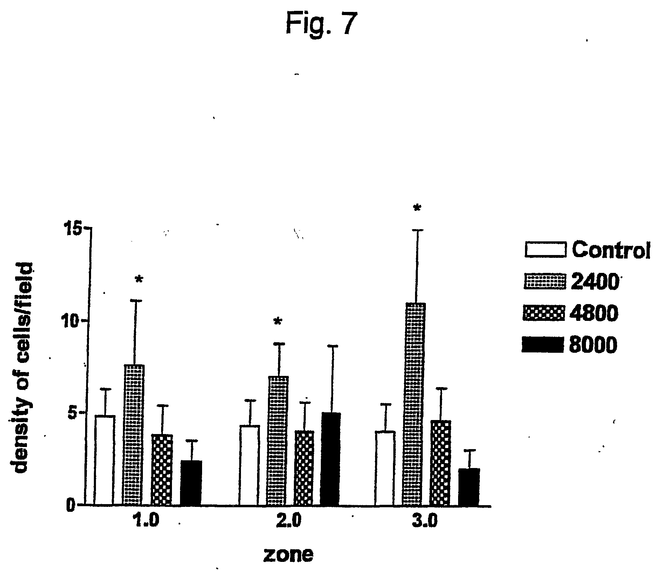

Fig. 7 represents the effect of increasing dose of Leukocyte Interleukin Injection (LI) treatment on the density of interleukin-2, receptor-positive (CD-25) lymphoid cells in oral squamous cell carcinoma (OSCC) (morphometry) on the Intraepithelial density. Zone 1.0 = tumor surface; Zone 2.0 = tumor center; Zone 3.0 = tumor-stroma interface. Data are presented as mean values ± SEM (n=27, control group; n=11, LI-treated group) (**P* < 0.05).

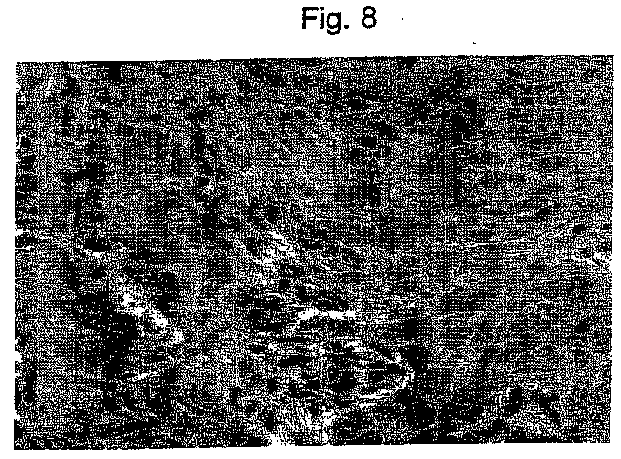

Fig. 8 represents a control case of oral squamous cell carcinoma on the density of T cells; immuno-localization of CD3 - positive cells within the control group (case 10) in this trial (original magnification X400).

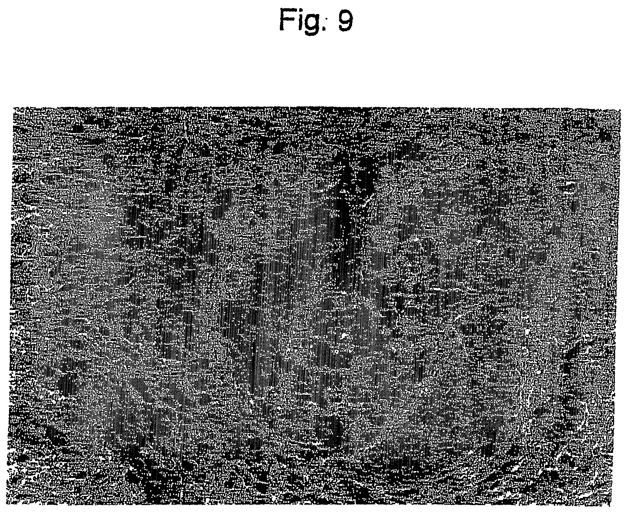

Fig. 9 represents the effect of Leukocyte Interleukin Injection (LI) treatment of oral squamous cell carcinoma on the density of CD3-positive T cells; immuno-localization of CD3 - positive cells of LI treated (case 31 having an original magnification X400).

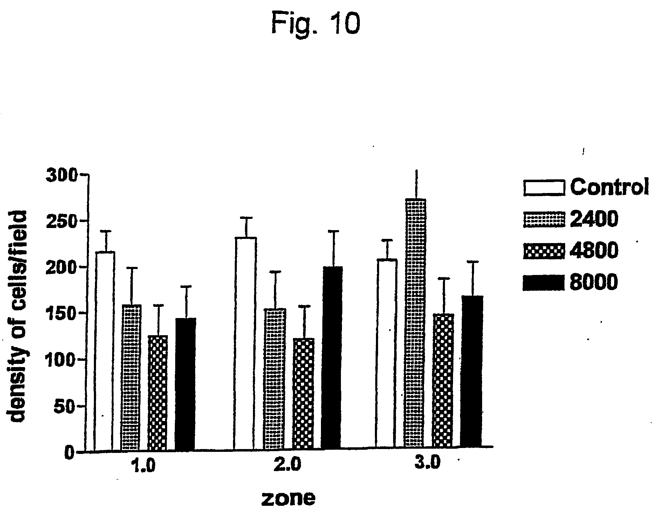

Fig. 10 represents the effect of increasing dose of Leukocyte Interleukin Injection (LI) treatment on the density of CD3-positive T cells in oral squamous cell carcinoma (OSCC) morphometry with respect to stromal density. Zone 1.0 = tumor surface; Zone 2.0 = tumor center; Zone 3.0 = tumor-stroma interface. Data are presented as mean values ± SEM (n=27, control group; n=25, LI-treated group) (**P* < 0.05).

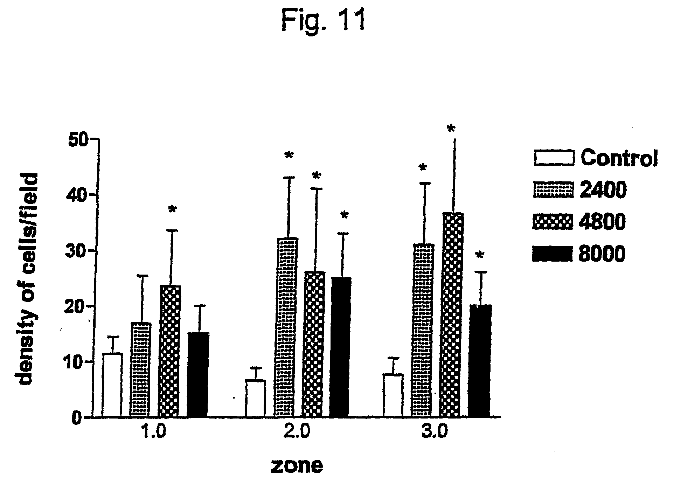

Fig. 11 represents the effect of increasing dose of Leukocyte Interleukin Injection (LI) treatment on the density of CD3-positive T cells in oral squamous cell carcinoma (OSCC) morphometry with respect to tumor intraepithelial density. Zone 1.0 = tumor surface; Zone 2.0 = tumor center; Zone 3.0 = tumor-stroma interface. Data are presented as mean values ± SEM (n=27, control group; n=25, LI-treated

### DETAILED DESCRIPTION OF THE INVENTION AND PREFERRED

### EMBODIMENTS

The present invention is concerned with use of a novel serum-free and mitogen-free cytokine mixture comprised of specific ratios of IL-I β to IL-2, TNF-α to IL-2, IFN-γ to IL-2 and GM-CSF to IL-2 for pre-sensitizing OSCCT. One such novel cytokine mixture is Leukocyte Interleukin Injection (LI) or Multikine®, which has demonstrated immuno-modulatory capabilities. The clinical significance of the immuno-suppression in OSCCT patients unexpectedly impacts methods of pre-sensitizing cancer prior to therapeutic treatment and in particular entry of the tumor cell into a cell cycle phase.

Immune restoration of head and neck cancer patients is accomplished by the infusion of cytokines such as IL-2, IFN α-γ or IL- 12. Whiteside, "Immunobiology and immunotherapy of head and neck cancer", Curr Oncol Rep 2001 ;3:46-55. In head and neck cancer, interleukin-based cytokine therapy resulted in immuno-augmenting. Cortesina G et al., "Interleukin-2 injected around tumor-draining lymph nodes in head and neck cancer", Head Neck 1991; 13:125-31; De Stefani et al., "Treatment of oral cavity and oropharynx squamous cell carcinoma with perilymphatic interleukin-2: clinical and pathologic correlations", J Immunother 1996;19:125-33; Valente et al., "Infiltrating leukocyte populations and T-lymphocyte subsets in head and neck squamous cell carcinomas from patients receiving perilymphatic injections of recombinant interleukin 2", Mod Pathol 1990;3:702-8; Whiteside TL et al., "Evidence for local and systemic activation of immune cells by peritumoral injections of interleukin 2 in patients with advanced squamous cell carcinoma of the head and neck", Cancer Res 1990;53:5654-62; Barrera et al., "Combination immunotherapy of squamous cell carcinoma of the head and neck", Arch Otolaryngol Head Neck Surg 2000;126:345-51; Verastegui et al., "A natural cytokine mixture (IRX-2) and interference with immune suppression induce immune mobilization and regression of head and neck cancer", Int J Immunopharmacol 1997;19:619-27; Hadden et al., "Interleukins and contrasuppression induce immune regression of head and neck cancer", Arch Otolaryngol Head Neck Surg 1994;120:395-403. For example, human (r)hIL-2 was successfully used to improve immune function of head and neck cancer patients as measured by cytotoxic T lymphocyte [CTL] and delayed type hypersensitivity [DTH] responses. The decreased response of T cells was shown by the decreased expression of the T cell receptor (TCR), its key signaling components, the ξ chain and zap-70, the absence of IL-2 production and increased apoptosis of T cells. Whiteside TL., "Immunobiology and immunotherapy of head and neck cancer", Curr Oncol Rep 2001;3:46-55. Studies investigating the causes of the impaired T cell function in head and neck cancer showed that the Fas-FasL system, TGF-β and PGE₂ are expressed at high levels.

However, *in vivo* administration of rIL-2 increased density of CD25⁺ cells as well as natural killer (NK) cells, human leukocyte antigen (HLA)-DR⁺ lymphocytes and T cells. In another series of studies, positive clinical responses have been observed when a cytokine mixture was administered perilymphatically or peritumorally. However, none of these studies correlated an increased immune response with definitive follow-on treatment such as surgery, radiation therapy and/or chemotherapy.

### The Technology

Multikine® or Leukocyte-Interleukin Injection (LI), is a serum-free, mitogen-free, antibiotic-free preparation produced from human peripheral blood mononuclear cells that include T-cells, B cells and macrophages. There are three "families" of cytokines in Leukocyte Interleukin Injection (LI) or Multikine® that together impart the unique biological activity of Leukocyte Interleukin Injection (LI) or Multikine®. They include direct cytotoxic/cytostatic and virocidal/virostatic cytokines such as TNF-α, and IFN-γ, lympho-proliferative cytokines such as IL-1, and IL-2 and chemotactic cytokines such as IL-6, IL-8 and MIP-1α. Furthermore, the different cytokine and small biological molecules that constitute Leukocyte Interleukin Injection (LI) or Multikine® are all derived from the lectin (*e.g.* PHA) *in vitro* stimulation of human peripheral blood mononuclear cells that include T cells, B cells, and macrophages. Centrifugation on a Ficoll-Paque gradient separates the white blood cells (including T cells, B cells, and macrophages) from donor whole blood, and a series of washes (in physiologically buffered media) facilitates the isolation of lymphocytes, and the removal of red blood cells, cellular debris and other unwanted cellular components from the isolated white cell component of the whole donor blood.

Leukocyte Interleukin Injection (LI) or Multikine® contains different cytokines present at specific ratios of each cytokine to Interleukin 2 (IL-2) as follows: It-1β to IL-2 at a ratio range of 0.4 - 1.5, and preferably at 0.7+/- 0.1 (IL-1β/IL-2), TNF-α to IL-2 at a ratio range of 3.2 -11.3, and preferably at 9.5+/- 1.8 (TNF-α/IL-2), IFN-γ to IL-2 at a ratio range of 1.5 - 10.9, and preferably at 6.0+/- 1.1 (IFN-γ/IL-2), and GM-CSF to IL-2 at a ratio range of 2.2 - 4.8, and preferably at 4.0+/- 0.5 (GM-CSF/IL,-2).

The remainder of the different cytokines and other small biologically active molecules in Leukocyte Interleukin Injection (LI) or Multikine® are also present within each preparation of the small biologically active molecule to IL-2 is as follows: IL-3 to IL-2 in a ratio range of 0.38 - 0.68, preferably at 0.53+/- 0.15, IL-6 to IL-2 in a ratio range of 37.2 - 53.8, preferably at 46+/- 5.9, IL-8 to IL-2 in a ratio range of 261 - 561.5, preferably at 411+/- 10.6, IL-1α to IL-2 in a ratio range of 0.56 - 0.94, preferably at 0.75+/- 0.19, IL-10 to IL-2 in a ratio range of 2.82 - 3.22, preferably at 3.0+/- 0.18, IL-16 to IL-2 in a ratio range of 1.16 - 2.84, preferably at 1.84+/-0.68, G-CSF to IL-2 in a ratio range of 2.16 - 3.78, preferably at 2.97+/- 0.81, TNF-β to IL-2 in a ratio range of 1.17 - 2.43, preferably at 1.8+/- 0.63, MIP-1α to IL-2 in a ratio range of 15.7 - 37.16, preferably at 22.7+/- 7.0, MIP-1β to IL-2 in a ratio range of 17.1 - 28.5, preferably at 22.8+/- 5.7, a RANTES to IL-2 in a ratio range of 2.3 - 2.7, preferably at 2.5+/- 0.13, a EGF to IL-2 in a ratio range of 0.267 - 0.283, preferably at 0.275+/- 0.008, PGE₂ to IL-2 in a ratio range of 3.63 - 5.42, preferably at 4.5+/- 0.87 and TxB₂ to IL-2 in a ratio range of 23.47 - 25.13, preferably at 24.3+/- 0.83.

Leukocyte Interleukin Injection (LI) or Multikine® was tested using a characterization protocol and does not contain the following cytokines and other small biologically active molecules: IL-4, IL-7, and IL-15, TfR, sICAM, PDGF-AB, IFN-α, EPO, LTC 4, TNF-β2, FGF basic, Angiogenin, sE-selectin, SCF, and LIF. Leukocyte Interleukin Injection (LI) or Multikine® contains only trace quantities (just above the level of detection of the assay) of IL-12, and LTB 4.

In the manufacturing process, mononuclear cells are separated from human donor "buffy coats" by step-gradient centrifugation and cultured with PHA to enhance production and secretion of IL-2 and other cytokines from the donor white blood cells in culture as disclosed in U.S. Patents 5,093,479, 4,390,623, 4,388,309, 4,406,830, 4,661,447, 4,681,844 and 4,464,355, all of which are incorporated herein by reference. Subsequently, the culture supernatant is aseptically harvested, clarified and subjected to a commercial virus exclusion process. The supernatant is then further concentrated approximately 10 fold by ultrafiltration and microfiltration.

At this point, Human Serum Albumin, Inj. USP is added and the concentrate is then buffered to a physiological pH and brought to a target IL-2 concentration per the label claim (example 400 IU/mL). The concentrate is then subjected to a second micro-filtration (0.22 micron-rated filter) and aseptically dispensed into sterile serum-type vials and labeled by its IL-2 content. Product potency is measured by the incorporation of radio-labeled thymidine by a cytotoxic T-lymphoid line (CTLL-2). The final injectable agent is further tested by ELISA for the presence of five marker cytokines: IL-2, IL-1β, GM-CSF, IFN-γ, and TNF-α.

Leukocyte Interleukin Injection (LI) or Multikine® is provided frozen in a borosilicate glass serum vial containing 2.2 mL of drug at the label claim as IL-2 (400 IU/ml) for peritumoral, intratumoral, perilymphatic or subcutaneous administration. Leukocyte Interleukin Injection (LI) or Multikine® is subjected to quality control tests for identity, sterility, bacterial endotoxins, pH, and total protein concentration. Each vial is inspected for particulate contamination and appearance. The preparation has a total protein content of approximately 3 mg/mL (or +/- 1mg/mL) wherein the material is supplied sterile and pyrogen free. Leukocyte Interleukin Injection (LI) or Multikine® has an assigned expiration date of 24 months from date of manufacture when the drug is stored at -20°C.

### Definitions

IL-2 - Interleukin 2 (IL-2): A 15.5-kD glycoprotein synthesized by CD4+ helper T lymphocytes (Formally known as T cell Growth Factor). IL-2 has an autocrine effect acting on the CD4+ T lymphocytes that produce it and on other cells of the immune system (including B lymphocytes, CD8+ T lymphocytes, NK [Natural Killer] cells and others).

IL-1β - Interleukin 1 beta (IL-1β): A 17-kD cytokine synthesized by activated mononuclear phagocytes, is found in free form in the circulation and mediates inflammatory responses. It acts on CD4+ T lymphocytes to help facilitate their proliferation, and acts on B-lymphocytes as a growth and differentiation factor. It also induces the synthesis of IL-6 by mononuclear phagocytes.

TNF-α - Tumor Necrosis Factor alpha (TNF-α): A 157 amino acid (aa) residues protein, synthesized by stimulated monocytes, macrophages, B lymphocytes, T lymphocytes, an NK cells among others, found in a trimmeric form in the circulation. TNF mediates direct anti-tumor action, causing tumor cell lysis, facilitates leukocyte recruitment, inducing angiogenesis and promotes fibroblast proliferation.

IFN-γ- Interferon Gamma (IFN-γ): A 21-24-kD glycoprotein homodimer synthesized by activated T lymphocytes and NK cells, is a powerful activator of monocytes increasing monocytes ability to destroy intracellular microorganisms and tumor cells. It has direct anti-viral and anti-proliferative activity, and causes many cell types to express Class II MHC (Major Histocompatibility Complex) cell surface molecular complex, as well as increasing the expression of Class I MHC.

GM-CSF - Granulocyte Macrophage - Colony Stimulating Factor (GM-CSF): A 127 aa protein found as a monomer in the circulation, produced by macrophages and T lymphocytes, fibroblast and endothelial cells. It is a growth factor for hemopoietic cells, and stimulates the growth and differentiation of myelomonocytic lineage.

IL-3 - Interleukin - 3 (IL-3): A 20-kD Lymphokine synthesized by activated CD4+ T helper lymphocytes, acts as a colony-stimulating factor by facilitating the proliferation of some hematopoietic cells and promoting the proliferation and differentiation of T lymphocytes.

IL-6 - Interleukin - 6 (IL-6): A 26-kD cytokine produced by activated T lymphocytes, mononuclear phagocytes, endothelial cells, and fibroblasts. It acts on many cells but has a special function in enabling activated B-lymphocytes to differentiate into antibody secreting plasma cells, and induces hepatocytes to form acute-phase proteins (implicated in inflammatory responses) as well as fibrinogen.

IL-8 - Interleukin - 8 (IL-8): An 8-kD protein produced by macrophages and endothelial cells. Is a powerful chemotactic factor for neutrophils and T lymphocytes, and facilitates neutrophil adherence to endothelial cells.

IL-1α - Interleukin 1 alpha (IL-1α): A 17-kD cytokine (like IL-1β) is cleaved from a 33-kD precursor molecule, synthesized by activated mononuclear phagocytes, is rarely found in free form in the circulation and acts as a membrane-associated substance. It assists IL-1β in mediating inflammatory responses.

IL-10 - Interleukin - 10 (IL-10): An 18-kD polypeptide produced by CD4+ and CD 8+ T lymphocytes, monocytes, macrophages, activated B lymphocytes, and keratinocytes. It inhibits macrophages ability to present antigen particularly to T_{H}1-type cells, and secrete IL-6 and TNF.

IL-16 - Interleukin - 16 (IL-16): A 14-kD tetrameric protein produced by CD8+ T lymphocytes, eosinophils, mast cells and respiratory epithelial cells. It has strong chemoattraction properties for CD4+ T lymphocytes and monocytes.

G-CSF - Granulocyte Colony Stimulating Factor (G-CSF): A 22 - 25-kD homodimer glycoprotein produced by macrophages, endothelial cells, fibroblasts and stromal cells. It increases granulocyte progenitor cells in the marrow, and sustains increase in blood neutrophils. It also enhances the ability of neutrophils to exhibit enhanced super-oxide production thought to be important in the destruction of microbially infected cells and tumor cells.

TNF-β - Tumor Necrosis Factor beta (TNF-β): A 25-kD protein produced by activated lymphocytes. It can kill tumor cells in culture, and stimulates proliferation of fibroblasts. In addition it mimics most of the other actions of TNF-α.

MIP-1α - Macrophage Inflammatory Protein - 1 alpha (MIP-1α): A 66-aa monomeric protein produced by macrophages and other cells. It is a chemo-attractant for monocytes, T lymphocytes and eosinophils.

RANTES - An 8-kD protein produced by T lymphocytes and is a chemo-attractant to monocytes, T lymphocytes and eosinophils, and promotes inflammation.

EGF - Epidermal Growth Factor (EGF): A trisulfated polypeptide of 53-aa residues. EGF is a member of the tyrosin kinase family, and has multiple functions including stimulation of the mitogenic response and assisting in wound healing.

PGE₂ - Prostaglandin E₂ (PGE₂): PGE₂ belong to a family of biologically active lipids derived from arachidonic acid through the cyclooxygenase enzymatic reaction. It is released by activated monocytes and blocks MHC Class II expression on T lymphocytes and macrophages.

TxB₂ - Thromboxane B₂ (TxB₂): TxB₂ is a member of biologically active compounds derived from polyunsaturated fatty acids by isomerization of prostaglandin and endoperoxidase PGH₂ via the enzyme thromboxane synthetase. TxB₂ has a physiological role in thromboembolic disease, and anaphylactic reactions.

CD25⁺ Cells - CD25 is a single chain glycoprotein, often referred to as the α-chain of the Interleukin-2-receptor (IL-2R) or the Tac-antigen that has a mol wt of 55 kDa and is present on activated T and B cells and activated macrophages. It functions as a receptor for IL2. Together with the β-chain of the IL-2R, the CD25 antigen forms a high-affinity receptor complex for IL-2.

CTLL-2 (Cell Line) - A line of mouse cytotoxic T lymphocytes obtained from C57B1/6 mice. This T cell line is dependent on an exogenous source of IL-2 for growth and proliferation.

Fas - FasL-The Fas/Fas Ligand system. The combination of a Fas antigen, a cell surface transmembrane protein that mediates apoptosis, and a complementary Fas-activated cytokine on a neutrophil that transduces an apoptotic signal into cells. Fas is a type-I membrane protein belonging to the tumor necrosis factor (TNF) receptor superfamily, and FasL is a member of the TNF family. FAS ligand is a membrane-bound protein of 31 kDa [kilo Dalton] (278 amino acids). The Fas-Fas ligand system plays important roles in many biological processes, including the elimination of autoreactive lymphoid cells. The Fas ligand is predominantly expressed in activated T lymphocytes and is one of the major effector molecules of cytotoxic T lymphocytes and natural killer cells.

HLA-DR⁺ Lymphocytes - Lymphocytes containing human leukocyte antigen (HLA)-DR antigens, a group of polymorphic glycoproteins determined by a glue sequence found in a leukocyte loci located on chromosome 6, the major histocompatibility loci in humans.

IU (International Units) - A unit of measure of the potency of biological preparations by comparison to an international reference standard of a specific weight and strength e.g., WHO 1^{st} International Standard for Human IL-2, 86/504. International Units are the only recognized and standardized method to report biological activity units that are published and are derived from an international collaborative research effort.

U (Units as a measure of biological activity) - Shorthand for a variety of named "units", which each laboratory derives as a reference, which is further unique to the laboratory where the work is being performed. Each "unit" is different from one laboratory to another laboratory and is not a globally recognized standard such as International Units (IU).

Mononuclear Infiltrate - Presence of monocytes, plasma cells, and lymphocytes, in tissue where they "normally" would not be present; or the presence of these cells in large numbers or abundance in clusters where they would otherwise be present in only a small number.

TCR ξ Chain - T-cell receptor-zeta chain. The zeta subunit is part of the TCR complex and is targeted towards the interaction of the TCR cell surface receptor with its ligand (antigen). The zeta subunit extending into the cell cytoplasm (cytosol) is phosphorylated at its tyrosine residues upon T cell activation and is implicated in signal transduction after TCR ligation.

TIL (Tumor Infiltrating Lymphocytes) - T lymphocytes isolated from the tumor they are infiltrating. Tumor Infiltrating lymphocytes have little or no cytotoxicity. TILs include CD4+ CD8+ predominantly T cells, and can be expanded *in vitro* by culture in the presence of IL-2. These cells are activated by the treatment with IL-2 and are frequently more aggressive towards the tumor from which they were isolated than normal lymphokine activated cells. The cytotoxic activity of TILs can be enhanced by IFN-γ. The antitumor activity of TILs *in vivo* can be blocked by TNF-β.

ZAP 70 - A 70 kD Zeta Associated Protein associated with the TCR ξ Chain that is a tyrosine kinase present in cytosol. ZAP 70 is thought to participate in maintaining T lymphocyte receptor signaling, mediating the signal transduction which eventually produces IL-2. The ZAP70 gene is expressed in T-cells and natural killer cells and maps to human chromosome 2q12.

ξ (Zeta) Chain - See TCR ξ Chain- The zeta chain gene is located on chromosome 1 in humans. The extracellular domain of this protein is nine amino acids long whereas the transmembrane domain contains a negatively charged aspartic acid residue and the cytoplasmic domain is 113 amino acids long. The cytoplasmic tail contains three of the antigen recognition motifs found in the cytoplasmic tails of CD3 chains. The zeta chain is also associated with other receptors such as the Fc (fragment, crystalline)-gamma receptor of NK cells.

USP - U.S. Pharmacopeia Monographs.

*P -* "p < 0.01": A term in mathematical statistics that denotes the level of probability of an event occurring under pre-set conditions.

ANOVA (Analysis of Variances) - A single factor analysis as described in Statistics and mathematical textbooks e.g., "Handbook of Statistical Methods for Engineers and Scientists", Harrison M. Wadsworth, Jr., Ed., McGraw Hill 1990, and "Statistical Operations Analysis of Health Research Data", Robert P. Hirsch and Richard K. Riegelman, Eds. Blackwell Science Inc., 1996.

### Mode of action and characterization of Leukocyte Interleukin Injection (LI) or Multikine®

Leukocyte Interleukin Injection (LI) or Multikine® is a biologically active, minimally toxic, immunomodulatory mixture of naturally derived and naturally occurring human cytokines produced under set conditions as described herein. Leukocyte Interleukin Injection (LI) or Multikine® can be used as an anti-cancer and anti-viral therapy or as a neo-adjuvant therapy with a broad-spectrum application for cancer, infectious disease, and other diseases states responding to immunomodulation.

Leukocyte Interleukin Injection (LI) or Multikine® can be made by methods known within the art. Mizel et al., "Purification to Apparent Homogeneity of Murine Interleukin 1", J. Immunol. 126:834 (1981); Togawa et al., "Characterization of Lymphocyte-Activating Factor (LAF) Produced by Human Mononuclear Cells: Biochemical Relationship of High and Low Molecular Weight Forms of LAF", J. Immunol. 122: 2112 (1979); Lachman et al., "Purification of Human Interleukin 1", Chem. Abstr. 94: 137539t (1981) of J. Supramolec. Struct. 13: 457 (1980); Lachman et al., "Partial Purification of Human Lymphocyte Activating Factor", *Chem. Abstr.* 93: 165824e (1980) of Prep. Biochem. 10: 387 (1980); Mizel et al., "Characterization of Lymphocyte Activating Factor Obtained from the Murine Microphage Cell Line P388D1", *Chem. Abstr.* 93:93346a (1980), of Biochem. Charact. Lymphokines, Proc. Int. Lymphokine Workshop 2nd (1979) pp. 411-418; Economou et al., "Purification, Physicochemical Characterization and a Biological Role of Lymphocyte Activating Factor (LAF)", *Chem. Abstr.* 93: 93347b (1980) of Biochem. Charact. Lymphokines, Proc. Int. Lymphokine Workshop, 2nd (1979) pp. 419-421; Simon et al., "The Role of Subcellular Factors in Pulmonary Immune Function: Physicochemical Characterization of Two Distinct Species of Lymphocyte-Activating Factor Produced by Rabbit Alveolar Macrophages", J. Immunol. 126: 1534 (1981). Animal studies also demonstrated that "mixed interleukins" may have immunomodulatory and immunostimulatory activity *in vitro.* Hadden et al., "Mixed Interleukins and Thymosin Fraction V Synergistically Induce T Lymphocyte Development in Hydrocortisone-Treated Aged Mice", Cell. Immunol. 144:228-236 (1992). Without being limited to any one theory of invention, one possible hypothesis is that the local/regional injection of "mixed interleukins" such as Leukocyte Interleukin Injection (LI) or Multikine® overcomes local immuno-suppression. Subsequently, a break tolerance to tumor antigens occurs and allows for an effective local anti-tumor immune response to occur.

It has been shown that the local instillation of interleukins in the region of the tumor or the actual transfection of Interleukin genes into a tumor markedly augments the anti-tumor immune response resulting in tumor regression as reported by Golumbek et al., "Treatment of Established Renal Cancer by Tumor Cells Engineered to Secrete Interleukin-4", Science 254:713-716 (1991). However, none of these studies discovered the highly unexpected effect of inducing malignant cells into a cell cycle phase without causing the active proliferation of the tumor.

Unexpectedly, the administration of Leukocyte Interleukin Injection (LI) or Multikine® pre-surgery leads to an increase in the number of tumor cells in the cell cycle phase without increasing the risk of a more rapidly growing and more rapidly recurring tumor as would otherwise be predicted from the art. The ability to induce tumor cells into cell-cycle appears to be unique to Leukocyte Interleukin Injection (LI) or Multikine® and may be due to the synergistic effect of the different cytokines present in this investigational drug and the differential effect of these cytokines on both the host's immune system and the tumor cells.

Data regarding the recurrence rate of patients treated with Leukocyte Interleukin Injection (LI) or Multikine® prior to surgery showed no recurrence of cancer at 24 months post treatment with Leukocyte Interleukin Injection (LI) or Multikine®. Remarkably, a small cohort of 8 sequentially treated patients did not have a single recurring patient in the 24 months follow-up period. In stark contrast, the literature teaches that the recurrence rate of similar patients is about 50% at 18-24 months post surgery.

Leukocyte Interleukin Injection (LI) or Multikine® treatment does not induce active proliferation of tumor residing lymphoid cells. Correspondingly, stromal Ki-67⁺ cells decreased while the frequency of Ki-67⁺ cancer cells increased following Leukocyte Interleukin Injection (LI) or Multikine® treatment. Thus, Leukocyte Interleukin Injection (LI) or Multikine® treatment induces an increase in the number of cycling tumor cells leading to increased susceptibility of the residual tumor to follow-on treatment with radiation and/or chemotherapy. Although other studies conducted with both natural and recombinant cytokines have shown efficacy in the treatment of cancer therapy, those studies have failed to teach the induction of entry into the cell cycling phase or the new method of synergistically combining Leukocyte Interleukin Injection (LI) or Multikine® with chemotherapy, immuno-therapy and radiation therapy.

For example, studies with the use of natural human and recombinant IL-2 and other cytokines as well as in local-regional therapy demonstrated the immune augmenting and anti-cancer activity at various sites. In particular, IL-2 demonstrates activity in the pleural cavity, liver and the urinary bladder while IFN-α demonstrates activity in the ovary while IFN-β demonstrates activity in the brain as reported by Yasumoto et al., "Induction of lymphokine-activated killer cells by intrapleural instillations of recombinant interleukin-2 in patients with, malignant pleurisy due to lung cancer", Cancer Res 1987;47:2184-7; Mavilgit et al., "Splenic versus hepatic artery infusion of interleukin-2 in patients with liver metastases", J Clin Oncol 1990;8:319-24; Pizza et al., "Tumor regression after intralesional injection of interleukin-2 (IL-2) in bladder cancer. Preliminary report", Int J Cancer 1984;34:359-67; Berek et al., "Intraperitoneal recombinant α-interferon for "salvage" immunotherapy in stage III epithelial ovarian cancer: a gynecologic oncology group study", Cancer Res 1985;45:4447-53; and Fettel et al., "Intratumor administration of beta-interferon in recurrent malignant gliomas-A phase I clinical and laboratory study", Cancer 1990;65:78-83. Even further, IFN-γ has been shown to demonstrate activity in skin while TNF-α demonstrates activity in the genitalia while a mixture of various cytokines demonstrates activity in the head and neck as reported by Edwards et al., "The effect of intralesional interferon gamma on basal cell carcinomas", J Am Acad Dermatol 1990;22:496-500; Irie et al., "A case of vulva cancer responding to the recombinant human tumor necrosis factor (PT-950) local injection therapy", Gan No Rinsho 1988;34:946-50; and Pulley et al., "Intravenous, intralesional and endolymphatic administration of lymphokines in human cancer", Lymph Res 1986;5:S157-63. Moreover, studies of recombinant IL-2 administrations for 10 days prior to surgery in the jugular peri-lymphatic or jugular peri-lymphatic and under the chin showed variable necrosis and lymphocytic infiltration as reported by Valente et al., "Infiltration leukocyte polulations and T-lymphocyte subsets in head and neck squamous cell carcinomas from patients receiving perilymphatic injections of recombinant interleukin-2", Mod Pathol 1990;3:702-8 and DeStefani et al., "Treatment of oral cavity and oropharynx squamous cell carcinoma with perilymphatic interleukin-2: clinical and pathologic correlations", J Immunother 1996;19:125-33. Moreover, microscopic examination of the resected tumors demonstrated an increase in lymphocytic infiltrate correlating to the clinical observations of IL-2 as reported by Saito et al., "Immunohistology of tumor tissue in local administration of recombinant interleukin-2 in head and neck cancer", Nip Jibi Gakkai Kaiho 1989;92:1271-6.

Nevertheless, none of the aforementioned studies showed any change in the gross dimensions of resected tumors despite two remissions at putatively high doses of recombinant IL-2 (800,000 U for four weeks [U=Units]) in 20 head and neck cancer patients as reported by Saito et al., "Clinical evaluation of local administration of rIL-2 in head and neck cancer", Nip Jibi Gakkai Kaiho. 1989;921271-6. Furthermore, the aforementioned studies have been limited by the small number of patients and hampered by the lack of a pathological comparison to a control group. Vlock et al., "Phase Ib trial of the effect of peritumoral and intranodal injections of interleukin-2 in patient with advanced squamous cell carcinoma of the head and neck: an Eastern Cooperative Oncology Group trial", Immunother 1994;15:134-139; Musiani et al., "Effect of low doses of interleukin-2 injected perilymphatically and peritumorally in patients with advlmced primary head and neck squamous cell carcinoma", J Bioi Res Modi 1989;8:571-578.

Although a recent randomized multi-center phase III study of 202 OSCC patients by De Stefani et al. indicated that peri-lymphatic administration of low does (5000U/day [U=Units]) of recombinant human IL-2 for 10 days prior to surgery, into the ipsylateral cervical lymph node chain, resulted in a significant (p< 0.01) increase in disease-free survival, which in turn resulted in longer overall survival (p< 0.03), De Stefani et al. failed to assess the role of this treatment regimen on cell cycling and its effect on the improvement of radiation and chemotherapy. De Stefani et al., "Improved Survival With Perilymphatic Interleukin 2 in Patients With Resectable Squamous Cell Carcinoma of the Oral Cavity and Oropharynx", Cancer 2002; 95: 90-97. Furthermore, despite teaching 5000U/day, no comparisons between the present invention and De Stefani et al. could be made with regard to De Stefani et al.'s teaching of a "high" and "low" dose of an administered biologic because the drug potency was measured by an non-definable U (Units). In contrast, the present disclosure validated and completed the full USP analytical methods validation program for determining the biological activity of Leukocyte Interleukin Injection (LI) or Multikine® in IU (International Units).

### Methods

One skilled in the art will appreciate that tumor cell proliferation measured by an immunohistochemistry Ki-67 marker or other equivalent means such as through the use of PCNA marker, p53 marker can be used as a prognostic parameter, de Vicente et al., "Expression of cyclin D1 and Ki-67 in squamous cell carcinoma of the oral cavity: clinicopathological and prognostic significance", Oral Oncol 2002; 38: 301-8*;* Bettendorf et al., "Prognostic relevance of Ki-67 antigen expression in 329 cases of oral squamous cell carcinoma", ORL J Otorhinolaryngeol Relat Spec 2002;64:200-5*.* In conjunction, flow cytometry or conventional staining methods and the use of microscopy with clinical, histopathological and tumor staging and classification (TNM, Tumor, Node, Metastasis) are used with others to indicate the aggressiveness of the disease process. Kerdpon et al., "Expression of p53 in oral mucosal hyperplasia, dysplasia and squamous cell carcinoma", Oral Disease 1997;3:86-92*.*

A Ki67 cell proliferation marker differentiates and is specific for only cells in cell cycle stages. G₁ is the first growth phase; S is the second phase marked by the initiation of DNA synthesis by the cell where cellular DNA replicates, and G₂ the second growth phase of the cell follows DNA replication in which the cell doubles in size. M is the last phase in the cell cycle where mitosis occurs wherein the cell divides into a daughter cell from the original parent cell. Each resulting cell contains a complete replica of the DNA of the original parent cell. Ki67 cellular marker being specific to cells in the cell cycle cannot be found in cells that are in G₀, which is a resting phase of the cell. During G₀, the cell does not undergo cellular replication, proliferation or DNA replication. Notably, the cell cycle phase phenomenon is a property common to all living eukaryotic cells including tumor cells.

To detect tumor cell proliferation, the presence of Ki-67 in residual tumor cell nests following surgical excision is determined. Raybaud et al., "Nuclear DNA content, an adjunct to p53 and Ki-67 as a marker of resistance to radiation therapy in oral cavity and pharyngeal squamous cell carcinoma", Int J. Oral Maxillofac Surg 2000; 29:36-41*;* Koelbl et al., "p53 and Ki-67 as predictive markers for radiosensitiveity in squamous cell carcinoma of the oral cavity? An immunohistochemical and clinicopathologic study", Int J Radiat Oncol Biol Phys 2001; 49:147-54*.* Generally, Ki-67 can be found in cells undergoing cell cycle G₁, S, G₂, and M but not in "resting" tumor cells (G₀). Since cycling tumor cells are both more radio- and chemo- sensitive, and non-cycling tumor cells are by-and-large radio-and chemo-resistant.

Tumors from head and neck cancer patients treated with Leukocyte Interleukin Injection (LI) or Multikine® prior to surgical resection of the residual tumor were studied and analyzed by immuno-histopathology. Timár et al., "The effect of Leukocyte Interleukin, Injection on the peri- and intratumoral subpopulation of mononuclear cells and on tumor epithelia - A possible new approach to augmenting sensitivity to radiation and chemotherapy in oral cancer. A multi-center Phase I/[pi] clinical trial", The Laryngoscope 113, December 2003. The study was conducted in a blinded manner and executed by three independent qualified pathologists that were blinded to the treatment and patient population, treated or control. The clinical study analyzed a cohort of 54 oral squamous cell cancer patients (H&NC) as part of a phase I-II clinical trial. These patients were investigated for safety of the therapeutic regimen, tumor and clinical responses, and for the composition of the mononuclear infiltrate and cell cycling rates.

The treatment regimen in the present examples section for the pre-sensitization of cancer with Leukocyte Interleukin Injection (LI) or Multikine® is predicated on a treatment protocol developed for head and neck cancer patients proven in a statistically significant manner to significantly increase tumor cell cycling aimed at rendering these tumor cells more sensitive to follow on treatment with radiation and/or chemotherapy. The treatment included the administration of Leukocyte Interleukin Injection (LI) or Multikine® intradermally at the circumferential margins of the visible or palpable tumor mass.

A two-week course of ten (10) subcutaneous/subdermal daily injections of Leukocyte Interleukin Injection (LI) or Multikine® at a daily dose ranging from about 20 IU to 1600 IU as IL-2 was administered peritumorally at the circumferential margin of the tumor mass. Another course of treatment is in the range of 40 IU to 800 IU. Still another range is the range of 35 IU to 75 IU. Yet another specific non-limiting example of a suggested treatment contemplates administration of Leukocyte Interleukin Injection (LI) or Multikine® at a daily dose of 55 IU as IL-2 in a two-week course often (10) subcutaneous/subdermal daily injections.

### Patients

Information regarding the Leukocyte Interleukin, Injection (LI) treated and control patient-groups are provided in Tables 1 and 2. Fifty-four patients are included with twenty-seven patients in a control group and twenty-seven patients in a treatment group.

**Table 1**

| Leukocyte Interleukin, Injection Control Group: Patient Information | | | |
|---|---|---|---|
| Patient No. | Sex | Age (y) | Tumor localization |
| 1 | Female | 65 | F. Mouth |
| 2 | Male | 54 | Tongue |
| 3 | Male | 52 | Tongue |
| 4 | Male | 53 | Tongue |
| 5 | Male | 52 | Tongue |
| 6 | Male | 57 | Tongue |
| 7 | Male | 40 | Tongue |
| 8 | Male | 59 | Tongue |
| 9 | Male | 43 | F. Mouth |
| 10 | Male | 53 | Tongue |
| 11 | Male | 45 | Tongue |
| 12 | Female | 50 | Tongue |
| 13 | Male | 66 | Lip |
| 14 | Male | 75 | Lip |
| 15 | Male | 67 | Lip |
| 16 | Male | 55 | Tongue |
| 17 | Male | 58 | Tongue |
| 18 | Female | 53 | F. Mouth |
| 19 | Male | 46 | F. Mouth |
| 20 | Male | 71 | Tongue |
| 21 | Male | 61 | Tongue |
| 22 | Female | 43 | Tongue |
| 23 | Male | 75 | Lip |
| 24 | Male | 77 | Lip |
| 25 | Male | 76 | Ventral tongue |
| 26 | male | 61 | Ventral tongue |
| 27 | Male | 54 | Tongue |

| | | | |
|---|---|---|---|
| F. mouth = floor of mouth | | | |

**Table 2**

| Patient information for Leukocyte Interleukin injection (LI) Treatment group | | | |
|---|---|---|---|
| Patient No. | Sex | Age (y) | Tumor localization |
| High dose (HD) | | | |
| 24 | Male | 58 | Tongue |
| 29 | Male | 74 | Tongue |
| 27 | Male | 55 | Retromolar |
| 26 | Male | 43 | F. mouth |
| 31 | Male | 64 | Ventral tongue |
| 30 | Male | 45 | F. mouth |
| 21 | Male | 47 | Ventral tongue |

| Medium dose (MD) | | | |
|---|---|---|---|
| 25 | Male | 65 | Lip |
| 20 | Male | 46 | Ventral tongue |
| 07 | Male | 52 | F. mouth |
| 13 | Male | 56 | F. Mouth |
| 14 | Male | 50 | F. Mouth |
| 15 | Male | 64 | Tongue |
| 23 | Male | 49 | F. mouth |
| 17 | Male | 41 | Ventral tongue |
| 18 | Male | 51 | F. mouth |
| 06 | Male | 51 | Tongue |
| 12 | Female | 57 | Oropharynx |
| 22 | Male | 62 | F. mouth |

| Low dose (LD) | | | |
|---|---|---|---|
| 08 | Male | 62 | F. mouth |
| 04 | Female | 65 | Tongue |
| 09 | Female | 49 | Retromolar |
| 03 | Female | 63 | F. mouth |
| 01 | Male | 58 | Ventral tongue |
| 02 | Male | 62 | Tongue |
| 05 | Male | 55 | Ventral tongue |
| 11 | Male | 57 | Ventral tongue |

| | | | |
|---|---|---|---|
| HD= high cumulative dose of Leukocyte Interleukin, Inj (8000 IU, as IL-2 equivalent), MD= medium dose (4800 IU) LD= low dose (2400 IU) | | | |

### Treatment regimen with Leukocyte Interleukin Injection (LI) or Multikine® for cancer

Another embodiment contemplated by the present disclosure for a treatment regimen for the pre-sensitization of cancer with Leukocyte Interleukin Injection (LI) or Multikine® is predicated on a treatment protocol developed for head and neck cancer patients proven in a statistically significant manner to significantly increase tumor cell cycling aimed at rendering these tumor cells more sensitive to follow on treatment with radiation and/or chemotherapy. The treatment included the administration of Leukocyte Interleukin Injection (LI) or Multikine® subcutaneously in the area of the submandibular cervical lymph node chain.

A two-week course of ten (10) subcutaneous/subdermal daily injections of Leukocyte Interleukin Injection (LI) or Multikine® at a daily dose ranging from about 20 IU to 1600 IU as IL-2 was administered ½ peritumorally at the circumferential margin of the tumor mass, and ½ at the submandibular lymphatic chain ipsylateral to the tumor mass. Another course of treatment is in the range of 40 IU to 800 IU. Still another range is the range of 35 IU to 75 IU. Yet another specific non-limiting example of a suggested treatment contemplates administration of Leukocyte Interleukin Injection (LI) or Multikine® at a daily dose of 55 IU as IL-2 in a two-week course often (10) subcutaneous/subdermal daily injections.

Twenty-seven patients of the treatment group received peritumoral administration of Leukocyte Interleukin Injection (LI) or Multikine® in a dose escalating study. Leukocyte Interleukin Injection (LI) or Multikine® administrations were performed in the following manner: daily dose was injected peritumorally over a two-week period (3 times per week) at the following doses for each of the dose groups tested; low dose, 400 IU (International Units of IL-2) [IL-2-equivalent] daily (8 patients), medium dose, 800 IU (IL-2-equivalent) daily (12 patients), and 5 times per week at the high dose, 800 IU (IL-2-equivalent) daily (7 patients). All Leukocyte Interleukin Injection (LI) or Multikine® injections were administered intra-dermally at the circumferential margin of the visible/palpable tumor mass. Surgery aimed at resection of the residual tumor mass was performed between day 21 and day 28 following the initial administration of Leukocyte Interleukin Injection (LI) or Multikine®. Local/regional radiation therapy commenced in post-operative patients following wound healing at variable times post-surgery and was dependent on the individual patient recovery from the surgical intervention. Radiotherapy was generally initiated between two to four weeks post-surgery.

The administration of Leukocyte Interleukin Injection (LI) or Multikine® was preceded by the single intravenous infusion of cyclophosphamide, 300 mg/m² three-days prior to the first Multikine® administration. Indomethacin (25 mg) was self-administered orally (with food), three times daily, beginning 3 days post cyclophosphamide administration and until 24 hours prior to surgery. Zinc sulfate (50 mg) and multivitamin supplement, once daily, was self-administered beginning 3 days after cyclophosphamide administration and until 24 hours prior to surgery. These agents have no affect whatsoever on tumor cell cycling and were given at doses that are 3-5 fold below the normal cancer therapeutic doses for these drugs.

Leukocyte interleukin is filled and furnished by Chesapeake Biological Laboratories, Inc., Baltimore, MD, for CEL-SCI Corporation was provided frozen in a sealed borosilicate glass serum- type vial containing 2.2 mL of drug at 400 IU/mL (IL-2 equivalent) for peritumoral, intratumoral, perilymphatic, or sub-cutaneous administration. The preparation has a total protein content of approximately 3 mg/mL (or +/- 1 mg/mL). The material was supplied sterile and pyrogen free. The investigational drug has an assigned expiration date of 24 months from date of manufacture when the drug is stored at -20°C.

Cyclophosphamide USP (Bristol-Myers-Squibb, Morton, UK) was supplied as a sterile powder containing 45 mg sodium chloride, 75 mg mannitol, or approximately 82 mg sodium bicarbonate per 100 mg cyclophosphamide for reconstitution before intravenous infusion.

Indomethacin USP (Sanofi-Synthelabo, Paris, France) was supplied as 25-mg tablets for oral self-administration with food.

Zinc sulfate (50 mg) (R. P. Scherer Corporation, Clearwater, FL) and over-the-counter multivitamins were supplied by the clinic to each patient for self-administration.

Pathological studies were conducted by the Department of Tumor Progression, National Institute of Oncology, Budapest, Hungary (J.T.). A single pathology protocol governed the present study and described the preparation and fixation of the surgically excised specimens and the gross, macroscopic and microscopic examination, as well as histological and immuno-histochemistry procedures. Specimens were initially collected and processed by the pathology department at each participating institution, as detailed in the pathology protocol. The specimens were then shipped to the central pathology laboratory for accession, further processing, and pathological evaluation.

Diagnosis of the oral lesions was based on probe-excision biopsy of the suspected lesion, and cancers classified as T2-3N0-2M0 were selected for immunotherapy with the LI or Multikine® treatment regimen as described earlier. At the end of the LI or Multikine® treatment period and before surgery, clinical responses were evaluated and in accordance with tumor response patients were scheduled for tumor resection (surgical removal of the residual tumor), small excision biopsy, or no biopsy in the case of a complete clinical response. Accordingly, two forms of post-LI or Multikine® treatment pathology specimens were available for immuno-histochemical and pathology analysis: 1) complete tumors and 2) tumor biopsy specimens. The latter, because of their small size, limited the extent of pathological analysis of these samples.

### Histological Analysis

The excised tissue was placed in previously labeled containers with saline-buffered formaldehyde, fixed overnight before embedding the tissue in paraffin and preparing 5-µm slides for H&E staining and immuno-histochemical analysis. Histological analysis and American Joint Committee on Cancer grading were performed from H&E-stained sections. The histopathological analysis was performed on three different tumor regions: surface (zone 1.0), center (zone 2.0), and tumor-stroma interface (zone 3.0). Occurrence of necrotic tumor cells was also evaluated from H&E slides. The percentage of the epithelial component versus the stroma in the head and neck cancer tumors was determined by two methods. First, connective tissue was stained according to Mallory (trichome staining), and the slides were measured for the area of cancer nests (tumor epithelia) by using ImagePro analysis software (MediaCibemetics, Silver Spring, MD). Second, slides containing resected tissue were labeled for cytokeratin immuno-histochemically using pan-cytokeratin antibody from DAKO (Glostrup, Denmark) (A1A3+CK19), which labels cancer cells, and were examined microscopically and analyzed by the use of ImagePro analysis software.

### Determination of Proliferating Fraction of Cancer Cells

Paraffin sections were labeled with a mouse monoclonal antibody recognizing Ki-67 antigen (DAKO) to demonstrate the proportion of cycling cell population. Frequency of cycling tumor cells was counted at original magnification x 20 in three separate areas of the selected field. Furthermore, cycling stromal cells were also determined using the same criteria as for intraepithelial tumor cells. At least 3 x 100 tumor cells per area were evaluated.

### Characterization of Mononuclear Cell Infiltrate

Mononuclear cells present in the close vicinity of tumor cell nests were determined by immuno-histochemical analysis performed on paraffin sections of the tumor samples. Sections were deparaffinized and treated with microwave to retrieve antigenicity. Only commercial antibodies, which were previously demonstrated to consistently stain paraffin sections were used. Neutrophils were labeled using anti-myeloperoxidase antibody (mouse monoclonal, DAKO), and hemopoietic stem cells with mouse monoclonal anti-CD34 antibody (DAKO). Macrophage cell population was identified by the expression of CD68 antigen (mouse monoclonal anti-CD68, DAKO), and dendritic cells were identified by the expression of Cilia marker (mouse anti-Cilia, Immunotech, Paris, France). Lymphoid cells were identified by the expression of LCA antigen (mouse monoclonal anti-CD45, DAKO). B-cell population was labeled with mouse monoclonal anti-CD20 and T cells were identified by a rabbit polyclonal anti-CD3 antibody (both from DAKO). Cytotoxic T cells were identified by the expression of CD8 (by mouse monoclonal anti-CD8, DAKO), and NK cells were identified using the CD57 antigen (mouse monoclonal anti-CD57 antibody, Novocastra, Newcastle on Tyne, UK). Interleukin-2 receptor (IL-2R) expressing cells within the tumor epithelia and stroma were identified by using a mouse monoclonal antibody to IL-2R, CD25 (Novocastra). In all cases, appropriate isotype control antibody was used as negative control.

All immuno-histochemical labeling was performed with the DAKO LSAB-2 kit using a biotinylated anti-mouse/anti-rabbit immunoglobulin G linker and streptavidin-horseradish-peroxidase to reveal specifically bound antibodies. The Chromagen used was Amino-ethyl-carbazol (AEC) (red) label. Sections were counterstained for the nuclei with hematoxylin.

### Hot-Spot Consideration

The density of mononuclear cells was determined based on the "hot-spot" technique similar to measurements of microvascular density. In each studied tumor area, the density of infiltrating cells was measured at the region of the highest tumor infiltrating mononuclear cell density thereby minimizing extreme heterogeneity of the cellular infiltrates in tissues.

### Pathological Study Design

Tumor biopsy specimens from the LI-treated or Multikine® and control (non-LI-treated) groups were evaluated with H&E staining using microscopic appearance of the tumor. CD3, CD8, Cilia, and CD25 labeling was performed provided the size of the sample allowed the performance of all four labeling procedures. In the control group and in the LI-treated group, in which complete tumor removal was performed, the entire tumor tissue was available for analysis; therefore, the complete analytical program was employed. An original magnification x40 was used to select the slides for viewing and original magnification X100 during histological analysis of the selected areas of the slides.

The histological evaluation of each patient's tumor section was performed by three independent pathologists. Consensual determinations were reached where disagreement between pathologists existed. Morphometric measurements were performed by the same three pathologists without the knowledge of the clinical background and treatment outcome of each of the cases (LI-treated or control cases).

### Statistical Analysis

The data were analyzed by ANOVA single-factor analysis, and a p value of less than 0.05 (at α = 0.05) was considered to be statistically significant.

### Results

### Histological evaluation

The control group of OSCC consisted of planocellular cancers with various keratinization grades (BRI-BR3), as determined by Broder's classification. Odell et al., "The Progostic Value of Individual Histologic Grading Parameters in Small Lingual Squamous Cell Carcinomas; The Importance of the Pattern of Invasion", Cancer 74: 789 (1994). The LI-treated group did not differ from the control group with respect to tumor type as shown in Tables III and IV. This was further confirmed by the cytokeratin immuno-staining patterns revealing highly heterogeneous expression of CK-19 or pan-CK in both tumor groups.

**Table 3**

| Histology, Control Group | | | | | | | |
|---|---|---|---|---|---|---|---|
| Patient No. | Diagnosis | Grade | Stage | Size of tumor (mm) | Necrosis | Necrosis type | Necrosis (%) |
| 1 | SCC | BR3 | PT2N0 | 15x8 | - | - | |
| 2 | SCC | BR1 | PT2N0 | 11x6 | - | - | |
| 3 | SCC | BR2 | PT2N0 | 22x10 | + | Field | 1.4 |
| 4 | SCC | BR1 | PT2N0 | 12x8 | + | Field | 8.3 |
| 5 | SCC | BR3 | PT2N1 | 12x7 | - | - | |
| 6 | SCC | BR3 | PT2N0 | 7x5 | + | Unicell. | |
| 7 | SCC | BR2 | PT2N0 | 11x9 | - | - | |
| 8 | SCC | BR3 | PT2N2 | 15x11 | + | Field | 44 |
| 9 | SCC | BR2 | PT2N0 | 10x5 | - | - | |
| 10 | SCC | BR3 | PT3N0 | 22x13 | + | Field | 2 |
| 11 | SCC | BR3 | PT2N0 | 10x5 | - | - | |
| 12 | SCC | BR3 | PT2N0 | 14x10 | - | - | |
| 13 | SCC | BR1 | PT2N0 | 14x10 | + | Microfocal | |
| 14 | SCC | BR1 | PT2N0 | 9x6 | - | - | |
| 15 | SCC | BR1 | PT3N0 | 24x12 | - | - | |
| 16 | SCC | BR3 | PT2N0 | 12x3 | - | - | |
| 17 | SCC | BR1 | PT2N0 | 18x12 | + | Field | 8.4 |
| 18 | SCC | BR3 | PT3N0 | 20x17 | - | - | |
| 19 | SCC | BR1 | PT2N0 | 13x8 | + | Microfocal | |
| 20 | SCC | BR3 | PT2N2 | 13x9 | - | - | |
| 21 | SCC | BR3 | PT2N0 | 17x10 | + | Field | 12 |
| 22 | SCC | BR3 | PT2N0 | 13x8 | - | - | |
| 23 | SCC | BR2 | PT2N0 | 14x9 | - | - | |
| 24 | SCC | BR1 | PT2N0 | 10x2 | - | - | |
| 25 | SCC | BR3 | PT2N0 | 14x4 | - | - | |
| 26 | SCC | BR1 | PT2N0 | 13x4 | + | Unicell. | |
| 27 | SCC | BR3 | PT2N0 | 14x6 | + | Unicell. | |

| | | | | | | | |
|---|---|---|---|---|---|---|---|
| SCC = Squamous cell carcinoma BR = Broder's grade (keratinization) Size = Highest width x highest depth in mm Superfic.: Superficial (at air-tumor interface only) Unicell.: Unicellular necrosis only Microfocal: Necrosis is present only in microscopic foci Field: Confluent zones | | | | | | | |

**Table 4**

| Histology and Pathology; Leukocyte Interleukin, Injection Treated Group | | | | | | | |
|---|---|---|---|---|---|---|---|
| Patient No. | Diagnosis | Grade | Stage | Size of tumor (mm) | Necrosis | Necrosis type | Necrosis (%) |
| High dose (HD) | | | | | | | |
| 24 | SCC | BR3 | PT3N0 | B | + | Unicell. | |
| 29 | SCC | BR1 | PT2N0 | 10x15 | + | Unicell. | |
| 27 | SCC | | PT2N1 | 1x1 | - | | |
| 26 | SCC | BR3 | PT3N0 | B | + | Unicell. | |
| 31 | dysplasia | | | | - | - | |
| 30 | SCC | BR1 | PT3N1 | 5x6 | - | - | |
| 21 | SCC | BR1 | PT2N0 | 10x15 | - | - | |

| Medium dose (MD) | | | | | | | |
|---|---|---|---|---|---|---|---|
| 25 | SCC | BR3 | PT3N0 | 15x7.5 | + | Field | 2 |
| 20 | SCC | BR3 | PT2N0 | 12x10 | + | Field | 10 |
| 7 | SCC | BR3 | PT2N1 | B | - | - | |
| 13 | SCC | BR1 | PT2N0 | B | - | - | |
| 14 | SCC | BR2 | PT1N0 | 6x5 | - | - | |
| 15 | SCC | BR2 | PT1N0 | 8x5 | + | Superfic. | |
| 23 | SCC | BR3 | PT3N2 | B | - | - | |
| 17 | SCC | BR3 | PT2N0 | 9x3 | - | - | |
| 18 | SCC | BR2 | PT2N0 | 15x13 | + | Unicell. | |
| 6 | SCC | BR3 | PT2N1 | 8x1 | + | Superfic. | |
| 12 | SCC | BR2 | PT2NO | 1x36 | - | - | |
| 22 | SCC | BR1 | PT3N1 | B | - | - | |

| Low dose (LD) | | | | | | | |
|---|---|---|---|---|---|---|---|
| 8 | SCC | BR2 | PT2N1 | 4x3 | + | Microfocal | |
| 4 | SCC | BR1 | PT2N0 | 9x5 | - | - | |
| 9 | SCC | BR3 | PT2N1 | 3x1.5 | - | - | |
| 3 | SCC | BR3 | PT2N1 | B | - | - | |
| 1 | SCC | BR3 | PT2N2 | 10x9 | + | Field | 10 |
| 2 | SCC | BR2 | PT2N0 | 14x8 | + | Superfic. | 2 |
| 5 | SCC | BR2 | PT2N0 | 14x3 | - | - | |
| 11 | SCC | BR3 | PT2N0 | B | - | - | |

| | | | | | | | |
|---|---|---|---|---|---|---|---|
| B = Excision biopsy only SCC = Squamous cell carcinoma BR = Broder's grade (keratinization) Size = Highest width x highest depth in mm Superfic.: Superficial (at air-tumor interface only) Unicell.: Unicellular necrosis only Microfocal.: Necrosis is present only in microscopic foci Field: Confluent zones HD= high cumulative dose of Leukocyte Interleukin, Inj. (8000 IU, as IL-2 equivalent) MD= medium dose (4800 IU) LD= low dose (2400 IU) | | | | | | | |

### Tumor Stroma

The "fragmentation" of cancer nests could not be confirmed despite previous reports by Hadden et al., "Interleukins and contrasuppression induce immune regression of head and neck cancer", Arch Otolaryngol Head Neck Surg 120:395 (1994); Verastegui et al., "A natural cytokine mixture (IRX-2) and interference with immune suppression induce immune mobilization and regression of head and neck cancer", Int J Immunopharmac 19:619 (1997); and Barrera et al., "Combination Immunotherapy of squamous cell carcinoma of the head and neck", Arch. (2000). However, stroma-rich tumors were identified in 8 of 27 in the control group and 6 of 17 in the LI-treated group. Specific labeling of the cancer nests and the tumor stroma with Mallory trichome stain helped to discriminate between these areas. The percentage of the epithelial nests in the control and LI-treated groups was determined by using morphometry. The analysis revealed no significant difference thereby suggesting that LI treatment did not affect the tumor-stroma ratio.

### Necrosis and Proliferation

The effect of LI treatment on tumor necrosis as shown in Tables 3 and 4 and proliferation of cancer cells (Fig. 2) using histological analysis and specific identification of cycling cells detected by the Ki-67 marker were also evaluated. Histological analysis identified field-necrosis or microfocal, surface or unicellular necrosis in both LI-treated and control groups as shown in Table 5. The absence of any kind of necrosis in OSCC was similar in all of the tumors from the different LI-treated groups studied (57%-63%) as shown in Table 5. Field necrosis measured in the range of greater than 1% of the tumor volume was also similar in the various tumor groups of Tables 3 and 4.

**Table 5**

| Effect of Leukocyte Interleukin Injection treatment on the presence of necrosis in oral squamous cell carcinoma | | | | | |
|---|---|---|---|---|---|
| Leukocyte Interleukin, Inj. (IU as IL-2) | None | Superficial | Unicellular | Microfocal | >1% of Volume (Field) |
| Control (Not treated) | 16/27 | 0/27 | 3/27 | 2/27 | 6/27 |
| 2400 | 5/8 | 0/8 | 0/8 | 1/8 | 2/8 |
| 4800 | 7/12 | 2/12 | 1/12 | 0/12 | 2/12 |
| 8000 | 4/7 | 0/7 | 3/7 | 0/7 | 0/7 |

Detection of cycling cells by Ki-67 expression identified cancer cells and stromal cells of host cells such as mononuclear cells, fibroblasts, endothelial cells is shown in Fig. 2 (x100 magnification). Morphometric analysis of the density of Ki-67-positive cancer cells indicated that LI treatment induced significant increase (P < 0.05) in cycling tumor cells except at the highest LI dose administered as shown in Fig. 4. On the other hand, the incidence of cycling host cells, found primarily in the stromal area of the tumor, decreased with the increasing LI dose (Fig. 4), and the effects were proved to be significant in case of the lowest and the highest doses (P < 0.05). These findings support the conclusion that treatment with Leukocyte Interleukin Injection (LI) or Multikine® treatment causes cancer cells to enter a cell cycle phase but does not cause the host immune cells or stromal cells to cycle. Accordingly, the present invention contemplates Leukocyte Interleukin Injection (LI) or Multikine® treatment to induce cell cycle entry of a high proportion of the tumor cell population based on the expression of Ki-67 antigen.

Data regarding the recurrence rate of patients treated with Leukocyte Interleukin Injection (LI) or Multikine® prior to surgery followed by radiation therapy or watchful waiting did not exhibit an increase in the recurrence rate at 24 months post treatment with Leukocyte Interleukin Injection (LI) or Multikine®. A small cohort of 8 sequentially Leukocyte Interleukin Injection (LI) or Multikine® treated patients did not have a single recurring patient in the 24 months follow-up period. In contrast, the literature pegs the recurrence rate of these patients at about 50% at 18-24 months post surgery.

Leukocyte Interleukin Injection (LI) or Multikine® treatment did not appear to induce active proliferation of tumor residing lymphoid cells, and correspondingly stromal Ki-67⁺ cells decreased, while the frequency of Ki-67⁺ cancer cells increased following Leukocyte Interleukin Injection (LI) or Multikine® treatment. Thus, LI or Multikine® treatment increases the number of cycling tumor cells leading to increased susceptibility of the residual tumor to follow-on treatment with radiation and/or chemotherapy.

### Mononuclear Infiltrates

Mononuclear infiltrates were evaluated in the stromal compartment and in the cancer nests defined as intraepithelial infiltrates. There were no clear-cut differences identified between the tumors resected from the control and LI-treated group using conventional H&E staining. The control group was also highly heterogeneous in this respect. In particular, certain tumors were characterized by a dense leukocytic infiltrate while others by a plasmocytic one. Still yet others were characterized by a lymphoid one.

Density of macrophages identified by the CD68 marker was measured intraepithelially and in the tumor stroma. The intraepithelial density of macrophages was comparable to that of the stroma. A relatively high density of intraepithelial macrophages was in the control tumors similar to ones treated with LI.

The density of myeloperoxidase-positive neutrophil leukocytes in OSCC was also determined by morphometry. These studies indicated that there was no statistically significant difference in stromal or intraepithelial neutrophil density following LI treatment.

Morphometric measurements were performed on three areas of OSCC. A tumor surface was defined as a zone 1.0. The center of the tumor was defined as a zone 2.0. At the tumor-stroma interface frequently called the invasive edge was defined as a zone 3.0. A significant difference in macrophages or connective tissue ratio was not observed for these areas. However, to characterize the lymphoid infiltrate in the OSCC, discrimination between these three zones was studied irrespective of the actual similarity or difference between the samples.

### Effect of Leukocyte Interleukin Injection Treatment on Dendritic Cells in Oral Squamous Cell Carcinoma

Dendritic cells were identified by the CD1a marker that revealed a rich infiltrate in the peritumoral normal epithelia. This pattern of rich infiltrate was unequivocally decreased in the cancer nests. There was no significant presence of dendritic cells identified in the tumor stroma in either the control or the LI-treated group. Intraepithelial dendritic cell infiltrate was heterogeneous in cancer cases irrespective of the treatment groups.

### Effect of Leukocyte Interleukin Injection Treatment on Lymphoid Cell Infiltrate in Oral Squamous Cell Carcinoma

Lymphoid cells were identified by the expression of leukocyte common antigen (1CA, CD45) marker in the various zones of OSCC indicating a markedly denser presence of lymphocytes in the tumor stroma than in the cancer cell nests in an approximately 1:10 ratio. There were no significant geographical differences observed between the various regions of OSCC from the surface to the invasive edge in either treatment group with respect to the lymphoid infiltrate. LI treatment induced an increasing trend in the stromal lymphoid infiltrate that was not statistically significant, except at the lowest dose studied as shown in Fig. 3. However, only the lowest dose of LI treatment induced significant (P < 0.05) increase in intraepithelial CD45 cells as shown in Fig. 5. The highest dose of 11 decreased the intraepithelial density of CD45 positive cells, although the changes were not statistically significant.

### Effect of Leukocyte Interleukin Injection Treatment on Interleukin-2 Receptor Alpha-Positive (CD25-Positive) Lymphoid Cells in Oral Squamous Cell Carcinoma

Approximately 10% of the stromal or intraepithelial lymphoid cells were found to express IL-2Ra in OSCC samples. There were no significant differences in the incidence of CD25-positve lymphoid cells in the various zones of LI-treated OSCC cases. Treatment with LI increased both the stromal except zone 1 surface and the intraepithelial incidence of CD25-positive lymphoid cells exclusively at the lowest LI dose (P < 0.05) as shown in Fig.'s 6 and 7.

### Effect of Leukocyte Interleukin Injection Treatment on Density of B Cells in Oral Squamous Cell Carcinoma

B-cells identified by the CD20 marker were found in the tumor stroma exclusively in all LI-treated groups. There was no difference in the density of B cells in the various zones in the control cases. Treatment with LI induced redistribution of B cells from the surface zone to the invasive edge. However, the trends were not statistically significant.

### Effect of Leukocyte Interleukin Injection Treatment on T Cells in Oral Squamous Cell Carcinoma

The T-cell subpopulation of the mononuclear infiltrate in OSCC was identified - by the CD3 marker. The intraepithelial density of T cells was below 5% of the stromal density of T cells in the tumors in the control group as shown in Fig. 8 (X400 magnification). However, there were large variations in the number and percentage of CD3-positive T cells among the different control patients. CD3-positive T cells were more prevalent in the LI-treated group as shown in Fig. 9 (X400 magnification).

In the LI-treated tumors the stromal density of CD3- positive T cells was lower by 30% to 40% in most tumor zones and LI doses studied as shown in Fig. 10. However, the differences were not statistically significant because of the individual variations as shown in Fig. 10. In contrast to findings in the tumor stroma, LI treatment induced significant increase (P < 0.05) in the intraepithelial T-cell density without a clear dependence on the dose of LI treatment as shown in Fig. 11.

In the control group, approximately 50% of the T cells found in the stroma of OSCC could be considered to be cytotoxic T cells, characterized by the CD8 marker. Similar to CD3-positive cells, LI treatment induced reduction in the incidence of CD8-positive cells in the OSCC stroma of typically, 30% - 40%. But the differences were not statistically significant. Approximately 10% of the stromal cytotoxic T cells were found intraepithelially in the treatment-naive control OSCC cases. Although LI treatment at various doses and primarily at the two lower doses induced some increase in the intraepithelial CD8 cells, this increase depended on the various zones in the tumors and on the individual case studied and was not statistically significant.

### Detection of Natural Killer Cells

NK cells infiltrating the oral cancers in the present study were not detected in the entire patient group. The failure to detect NK cells in OSCC was not due to technical failure of the immunoreaction because N-CAM (CD57) was successfully detected in nerve cells and degenerating muscle cells in the region adjacent to the tumor tissue studied.

### Detection of CD34-Positive Hematopoietic Stem Cells in Oral Squamous Cell Carcinoma

Previous reports indicated that OSCC tumors may contain CD34-positive stem cells in their stroma. Schmidt et al., "Mechanisms of immune suppression in patients with head and neck cancer: presence of CD34+ cells which suppress immune functions within cancers that secrete granulocyte-macrophage colony-stimulating factor", Clin Cancer Res 1:95 (1999); Young et al., "Mechanisms of immune suppression in patients with head and neck cancer: influence on the immune infiltrate of the cancer", Int J Cancer 67:333 (1996). However, CD34-positive mononuclear cells were not detected in the present study of 54 paraffin-embedded samples. This was not due to technical failure because CD34-positive endothelial cells in the tumor microvasculature were readily detected in all OSCC cases studied.

### Discussion

The data from the present pathological study of 54 patients with OSCC demonstrates that OSCC is an immunogenic tumor and that LI treatment induces lymphocytic infiltration into a tumor. As in other cancer types, there is a marked individual variability between tumor samples obtained from different patients with regard to the composition of the mononuclear infiltrate of OSCC suggesting that a separate analysis would be necessary to determine which of the components of the cellular infiltrate plays a significant role in disease prognosis or therapeutic response in OSCC.

Leukocyte Interleukin (LI) treatment has a specific effect on the composition of the mononuclear infiltrate of the OSCC. There was no effect seen on stromal, intraepithelial macrophages, neutrophil leukocytes, or antigen presenting cells such as CD1a-positive. On the other hand, the normal peritumoral epithelium contained the highest density of dendritic cells in both control and LI-treated groups. Without being limited to any single theory of invention, this suggests that OSCC may secrete a factor(s) that interferes with or inhibits antigen-presenting cell activity directly at the tumor site. Since patients with malignant melanoma have a similar dendritic cell distribution, this may be a general phenomenon of OSCC rather than being treatment specific. Treatment with LI at the doses provided herein did not have an influence on this feature of OSCC.

Leukocyte interleukin injection (LI) treatment with the lowest dose induced significant accumulation of lymphoid cells in cancer nests of OSCC without significant effect on stromal density. Furthermore, LI treatment at 400 IU per day, three times a week increased the density of CD25-expressing lymphoid cells in the tumor stroma and intraepithelially. The possibility exists of a feedback inhibition loop by the relatively high local concentration of natural IL-2 in the LI preparation.

Analysis of the B cell population of lymphoid cells indicated that stromal B cells were not affected significantly by the LI treatment and that B cells were not present intraepithelially. Analysis of a T-cell subset as a target of LI treatment did not produce conclusive results. Although LI treatment of OSCC induced a decreasing trend of stromal density for both CD3- positive and CD8-positive T cells, a significant increase (P < 0.05) in intraepithelial density following LI treatment was observed for CD3-positive cells independent of the LI dose. Without being limited to any particular theory of invention, it is noted that another T-cell subset such as CD4-positive T cells may be affected by LI treatment.

Leukocyte interleukin injection (LI) treatment has no effect on several features of OSCC such as the expression of cytokeratin and Broder's grade. There were also no changes in the tumor-stroma ratio following LI treatment. There was no difference between the control treatment-naive group and the LI treatment group in the incidence of necrosis macroscopic or microscopic forms of cancer nests at the end of the LI treatment cycle and surgical resection of the residual tumor.

LI treatment induced cell cycle entry of a high proportion of the tumor cell population based on the expression of Ki-67 antigen. The LI induced the OSCC tumor cells into cell cycle due to the synergistic effect of the different cytokines present in this investigational drug (which include IL-1-β, IL-2, TNF-α, IFN-γ, and GM-CSF) and the differential effect of these cytokines on both the host's immune system and the tumor.

A small cohort of eight sequentially LI-treated patients from one study center did not have a single patient with recurrence in the 24-month follow-up period. Because an increase in cycling of tumor cells presents a risk of a more rapidly growing and more rapidly recurring tumor, the recurrence rate in the LI-treated patients versus the control (non-LI-treated) group was studied. Preliminary data regarding the recurrence rate in patients treated with LI before surgery who had follow-up with either radiation therapy or watchful waiting did not exhibit an increase in the recurrence rate at 24 months after treatment regimen.

A recent randomized multicenter Phase III study of 202 patients with OSCC by De Stefani et al. indicated that perilymphatic administration of low does (5000 LI/day) of recombinant human IL-2 for 10 days before surgery into the ipsilateral cervical lymph node chain resulted in a significant (P < 0.01) increase in disease-free survival, which in turn resulted in longer overall survival (P < 0.03). However, the primary target population of the LI treatment is the CD3-positive T cell. Therefore, the LI treatment induced a shift of stromal infiltrating T cells toward tumor intraepithelial which is important for anti-tumor action and destruction.

Treatment with LI did not appear to induce active proliferation of tumor residing lymphoid cells. Correspondingly, stromal Ki-67-positive cells decreased whereas the frequency of Ki-67-positive cancer cells increased following LI treatment. Thus, LI treatment seems to induce the migration of committed anti-tumor T cells toward the cancer nest and increase the number of cycling tumor cells leading to increased susceptibility of the residual tumor to follow-up treatment with radiation therapy, chemotherapy or both.

### Drug Safety, Pilot Efficacy and Compositions

Leukocyte Interleukin Injection (LI) or Multikine® has been tested in over 190 Cancer, HIV, and HIV/HPV infected, patients with no severe adverse events related to LI or Multikine® administration as reported by Harris et al., "Immunologic approaches to the treatment of prostate cancer", Semin Oncol. 1999 Aug;26(4):439-7; Timár et al., "The effect of Leukocyte Interleukin, Injection on the peri- and intratumoral subpopulation of mononuclear cells and on tumor epithelia - A possible new approach to augmenting sensitivity to radiation and chemotherapy in oral cancer. A multi-center Phase I/II clinical trial", The Laryngoscope [113 December 2003]; Brown et al., "A Phase I Open-Label Study of Leukocyte Interleukin, Injection in HIV-I infected individuals: preliminary evidence for improved delayed-type hypersensitivity responses to recall antigens", Antiviral Therapy 5 (supplement) 18, 2000; Taylor et al., "Immunotherapy with Leukocyte Interleukin, Injection for human papilloma virus (HPV) induced cervical dysplasia in HIV patients", Annual Meeting of the International Society for Interferon and Cytokine Research, Cleveland, OH, October 2001; Taylor et al., "Immunotherapy with Leukocyte Interleukin, Injection for human papilloma virus (HPV) induced cervical dysplasia in HIV patients", 33rd SGO Conference, Miami, FL, March 2002 Multikine® was also shown to be safe in animal toxicological studies in mice, rats, guinea pigs and dogs. Furthermore, Multikine(R) was tested for and has demonstrated pilot efficacy in head and neck cancer and cervical dysplasia.

Leukocyte Interleukin Injection (LI) or Multikine® may further be used as a component of an immunomodulatory composition together with one or more pharmaceutically acceptable carriers or adjuvants, either prophylactically or therapeutically. When provided for use prophylactically, the immunomodulatory composition is provided in advance of any evidence of infection or disease. While it is possible for Leukocyte Interleukin Injection (LI) or Multikine® to be administered in a pure or substantially pure form, a pharmaceutical composition, formulation or preparation may also be used.

The formulations of the present disclosure, both for clinical and for human use, may comprise Leukocyte Interleukin Injection (LI) or Multikine® as described above together with one or more pharmaceutically acceptable carriers and, optionally, other therapeutic ingredients, especially therapeutic immunological adjuvants. The carrier(s) must be "acceptable" in the sense of being compatible with the other ingredients of the formulation and not deleterious to the recipient thereof.

In general, the formulations are prepared by uniformly and intimately bringing into association the active ingredient with liquid carriers or finely divided solid carriers or both, and then, if necessary, bringing the product into the desired formulation. The term "pharmaceutically acceptable carrier" as used herein refers to any carrier, diluent, excipient, suspending agent, lubricating agent, adjuvant, vehicle, delivery system, emulsifier, disintegrant, absorbant, preservative, surfactant, colorant, flavorant, or sweetener. The formulations may conveniently be presented in unit dosage form and may be prepared by any method well-known in the pharmaceutical art.

Formulations suitable for intravenous, intramuscular, subcutaneous, or intraperitoneal, nasal, etc. administration conveniently comprise sterile aqueous solutions of the active ingredient(s) with solutions which are preferably isotonic with the blood of the recipient. The compounds of the present disclosure may also be administered orally, parenterally, by inhalation spray, topically, rectally, buccally, vaginally or via an implanted reservoir in dosage formulations containing conventional non-toxic pharmaceutically-acceptable carriers, adjuvants and vehicles. The term parenteral as used herein includes subcutaneous, intravenous, intramuscular, intraperitoneally, intrathecally, intraventricularly, intrasternal and intracranial injection or infusion techniques.

Such formulations may be conveniently prepared by dissolving solid active ingredients in water containing physiologically compatible substances such as sodium chloride (e.g. 0.1-2.0M), glycine, and the like, and having a buffered pH compatible with physiological conditions to produce an aqueous solution and rendering the solution sterile. These may be present in unit or multi-dose containers, for example, sealed ampules or vials.

The compounds of the present disclosure may also be administered in the form of sterile injectable preparations, for example, as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parenterally-acceptable diluents or solvents, for example, as solutions in 1,3-butanediol. Among the acceptable vehicles and solvents that may be employed are water, Ringer's solution and isotonic sodium chloride solution. In addition, sterile, fixed oils are conventionally employed as solvents or suspending mediums. For this purpose, any bland fixed oil may be employed including synthetic mono- or di-glycerides. Fatty acids such as oleic acid and its glyceride derivatives, including olive oil and castor oil, especially in their polyoxyethylated versions are useful in the preparation of injectables. These oil solutions or suspensions may also contain long-chain alcohol diluents or dispersants.

The compounds of this disclosure may also be administered topically, especially when the conditions addressed for treatment involve areas or organs readily accessible by topical application including disorders of the eye, the skin, or the lower intestinal tract. Suitable topical formulations are readily prepared for each of these areas.

For topical application to the eye, or ophthalmic use, the compounds can be formulated as micronized suspensions in isotonic, pH adjusted sterile saline, or preferably, as solutions in isotonic, pH adjusted sterile saline, either with or without a preservative such as benzylalkonium chloride. Alternatively, the ophthalmic uses of Leukocyte Interleukin Injection (LI) or Multikine® may be formulated in an ointment such as petrolatum.

For topical application to the skin, the compounds can be formulated in a suitable ointment containing the compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, the compounds can be formulated in a suitable lotion or cream containing the active compound suspended or dissolved in, for example, a mixture of one or more of the following: mineral oil, sorbitan monostearate, polysorbate 60, cetyl esters wax, cetearyl alcohol, 2-octyldodecanol, benzyl alcohol and water.

The amount of active ingredient that may be combined with the carrier materials to produce a single dosage form will vary depending upon the host treated and the particular mode of administration. Some factors include the activity of the specific compound employed, the age, body weight, general health, sex, and diet of the patients; the time of administration, rate of excretion, drug combination, and the severity of the particular disease being treated and form of administration.

Pharmaceutical methods may also be employed to control the duration of action. Controlled release preparations may be achieved through the use of polymer to complex or absorb the peptide. The controlled delivery may be exercised by selecting appropriate macromolecules (for example, polyester, polyamino acids, polyvinyl, pyrrolidone, ethylenevinylacetate, methylcellulose, carboxymethylcellulose, or protamine sulfate) and the concentration of macromolecules as well as the methods of incorporation in order to control release.

For example, Leukocyte Interleukin Injection (LI) or Multikine® may be incorporated into a hydrophobic polymer matrix for controlled-release over a period of days. Such controlled-release films are well known to the art. Particularly preferred are transdermal delivery systems. Other examples of polymers commonly employed for this purpose that may be used in the present invention include non-degradable ethylene-vinyl acetate copolymer and degradable lactic acid-glycolic acid copolymers which may be used externally or internally. Certain hydrogels such as poly(hydroxyethylmethacrylate) or poly(vinylalcohol) also may be useful, but for shorter release cycles then the other polymer releases systems, such as those mentioned above.

Alternatively, instead of incorporating these agents into polymeric particles, it is possible to entrap these materials in microcapsules prepared, for example, by coacervation techniques or by interfacial polymerization, for example, hydroxy-methylcellulose or gelatin-microcapsules and poly(methylmethacrylate) microcapsules, respectively, or in colloidal drug delivery systems, for example, liposomes, albumin microspheres, microemulsions, nanoparticles, and nanocapsules or in macroemulsions.

To be effective therapeutically as central nervous system targets, Leukocyte Interleukin Injection (LI) or Multikine® should also readily penetrate the blood-brain barrier when peripherally administered. Compounds which cannot penetrate the blood-brain barrier can be effectively administered by an intraventricular route or other appropriate delivery system suitable for administration to the brain.

Leukocyte Interleukin Injection (LI) or Multikine® may also be supplied in the form of a kit, alone, or in the form of a pharmaceutical composition as described above. Administration of Leukocyte Interleukin Injection (LI) or Multikine® can be conducted by conventional methods. For example, Leukocyte Interleukin Injection (LI) or Multikine® can be used in a suitable diluent such as saline or water, or complete or incomplete adjuvants. Leukocyte Interleukin Injection (LI) or Multikine® can be administered by any route appropriate for immune system stimulation, such as intravenous, intraperitoneal, intramuscular, subcutaneous, nasal, oral, rectal, vaginal, and the like.

As noted above, Leukocyte Interleukin Injection (LI) or Multikine® may be for either a prophylactic or therapeutic purpose. When provided prophylactically, Leukocyte Interleukin Injection (LI) or Multikine® is provided in advance of any evidence or in advance of any symptom due to disease. When provided therapeutically, Leukocyte Interleukin Injection (LI) or Multikine® is provided at (or after) the onset of the disease or at the onset of any symptom of the disease. The therapeutic administration of Leukocyte Interleukin Injection (LI) or Multikine® serves to attenuate the disease and improves conventional treatment outcomes.

The disclosure being thus described, it will be obvious that the same may be varied in many ways.

## Claims

1. Use of a therapeutically active amount of a serum-free and mitogen-free cytokine mixture for the manufacture of a medicament for pre-sensitizing oral squamous cell carcinoma tumors prior to a therapeutic treatment,
wherein said cytokine mixture is comprised of specific ratios of IL-1β, TNF-α, IFN-γ and GM-CSF to Interleukin 2 (IL-2) as follows:
(a) IL-1β to IL-2 at a ratio range of 0.4 - 1.5;
(b) TNF-α to IL-2 at a ratio range of 3.2 - 11.3;
(c) IFN-γ to IL2 at a ratio range of 1.5 - 10.9; and,
(d) GM-CSF to IL-2 at a ratio range of 2.2 - 4.8,
wherein half of said serum-free and mitogen-free cytokine mixture is to be administered peritumorally at a circumferential margin of the tumor mass and the other half of said mixture is to be administered at the submandibular lymphatic chain ipsylateral to the tumor mass three times a week over a two week period in a range from abut 20 IU to 1600 IU wherein IU represent International Units for Interleukin-2 give in World Health Organization 1^{st} International Standard for Human IL-2, 86/504.

2. Use of a therapeutically active amount of a serum-free and mitogen-free cytokine mixture for the manufacture of a medicament for pre-sensitizing oral squamous cell carcinoma tumors prior to a therapeutic treatment,
wherein said cytokine mixture is comprised of specific ratios of IL-1β, TNF-α, IFN-γ and GM-CSF to Interleukin 2 (IL-2) as follows:
(a) IL-1β to IL-2 at a ratio range of 0.4 - 1.5;
(b) TNF-α to IL-2 at a ratio range of 3.2 - 11.3;
(c) IFN-γ to IL2 at a ratio range of 1.5 - 10.9; and,
(d) GM-CSF to IL-2 at a ratio range of 2.2 - 4.8,
wherein said serum-free and mitogen-free cytokine mixture is to be administered perilymphatically three times a week over a two week period in a range from about 20 IU to 1600 IU wherein IU represent International Units for Interleukin-2 given in World Health Organization 1^{st} International Standard for Human IL-2, 86/504.

3. The use of claim 1 or 2, wherein said therapeutic treatment is selected from the group consisting of chemotherapy, immuno-therapy and radiation therapy.

4. The use of claim 1, wherein half of said serum-free and mitogen-free cytokine mixture is to be administered peritumorally at a circumferential margin of the tumor mass and the other half of said mixture is to be administered at the submandibular lymphatic chain ipsylateral to the tumor mass three times a week over a two week period in a range from about 40 IU to 800 IU wherein IU represent International Units for Interleukin-2 given in World Health Organization 1^{st} International Standard for Human IL-2, 86/504.

5. The use of claim 1, wherein half of said serum-free and mitogen-free cytokine mixture is to be administered peritumorally at a circumferential margin of the tumor mass and the other half of said mixture is to be administered at the submandibular lymphatic chain ipsylateral to the tumor mass three times a week over a two week period at 400 IU wherein IU represent International Units for Interleukin-2 given in World Health Organization 1^{st} International Standard for Human IL-2, 86/504.

6. The use of claim 1, wherein half of said serum-free and mitogen-free cytokine mixture is to be administered peritumorally at a circumferential margin of the tumor mass and the other half of said mixture is to be administered at the submandibular lymphatic chain ipsylateral to the tumor mass three times a week over a two week period at 800 IU wherein IU represent International Units for Interleukin-2 given in World Health Organization 1^{st} International Standard for Human IL-2, 86/504.

7. The use of claim 1, wherein half of said serum-free and mitogen-free cytokine mixture is to be administered peritumorally at a circumferential margin of the tumor mass and the other half of said mixture is to be administered at the submandibular lymphatic chain ipsylateral to the tumor mass five times a week over a two week period at 800 IU wherein IU represent International Units for Interleukin-2 given in World Health Organization 1^{st} International Standard for Human IL-2, 86/504.

8. The use of claim 2, said serum-free and mitogen-free cytokine mixture is to be administered perilymphatically three times a week over a two week period in a range from about 40 IU to 800 IU wherein IU represent International Units for Interleukin-2 given in World Health Organization 1^{st} International Standard for Human IL-2, 86/504.

9. The use of claim 2, wherein said serum-free and mitogen-free cytokine mixture is to be administered perilymphatically three times a week over a two week period at 400 IU wherein IU represent International Units for Interleukin-2 given in World Health Organization 1^{st} International Standard for Human IL-2, 86/504.

10. The use of claim 2, wherein said serum-free and mitogen-free cytokine mixture is to be administered perilymphatically three times a week over a two week period at 800 IU wherein IU represent International Units for Interleukin-2 given in World Health Organization 1^{st} International Standard for Human IL-2, 86/504.

11. The use of claim 2, wherein said serum-free and mitogen-free cytokine mixture is to be administered perilymphatically five times a week over a two week period at 800 IU wherein IU represent International Units for Interleukin-2 given in World Health Organization 1^{st} International Standard for Human IL-2, 86/504.

12. The use of claim 1 or 2, wherein the cytokine mixture further comprises
(a) an IL-3 to IL-2 ratio in a range from 0.38 - 0.68, preferably at 0.53 +/- 0.15;
(b) an IL-6 to IL-2 ratio in a range from 37.2 - 53.8, preferably at 46 +/- 5.9;
(c) an IL-8 to IL-2 ratio in a range from 261 - 561.5, preferably at 411 +/- 10.6;
(d) an IL-1α to IL-2 ratio in a range from 0.56 - 0.94, preferably at 0.75 +/- 0.19;
(e) an IL-10 to IL-2 ratio in a range from 2.82 - 3.22, preferably at 3.0 +/- 0.18;
(f) an IL-16 to IL-2 ratio in a range from 1.16 - 2.84, preferably at 1.84 +/- 0.68;
(g) a G-CSF to IL-2 ratio in a range from 2.16 - 3.78, preferably at 2.97 +/- 0.81;
(h) a TNF-β to IL-2 ratio in a range from 1.17 - 2.43, preferably at 1.8 +/- 0.63;
(i) a MIP-1α to IL-2 ratio in a range from 15.7 - 37.16, preferably at 22.7 +/- 7.0;
(j) a MIP-1β to IL-2 ratio in a range from 17.1 - 28.5, preferably at 22.8 +/- 5.7;
(k) a RANTES to IL-2 ratio in a range from 2.3 - 2.7, preferably at 2.5 +/- 0.13;
(l) a EGF to IL-2 ratio in a range from 0.267 - 0.283, preferably at 0.275 +/- 0.008;
(m) a PGE₂ to IL-2 ratio in a range from 3.63 - 5.42, preferably at 4.5 +/- 0.87; and/or
(n) a TxB₂ to IL-2 ratio in a range from 23.47 - 25.13, preferably at 24.3 +/- 0.83.

## Patentansprüche

1. Gebrauch einer therapeutisch aktiven Menge einer serumfreien und mitogenfreien Zytokinmischung zum Herstellen eines Arzneimittels zur Vorsensibilisierung von oralen Plattenepithelkarzinomen vor einer therapeutischen Behandlung,
wobei die Zytokinmischung aus spezifischen Verhältnissen von IL-1β, TNF-α, INF-γ und GM-CSF zu Interleukin 2 (IL-2) wie folgt zusammengesetzt ist:
(a) IL-1β zu IL-2 in einem Verhältnisbereich von 0,4-1,5;
(b) TNF-α zu IL-2 in einem Verhältnisbereich von 3,2-11,3;
(c) IFN-γ zu IL-2 in einem Verhältnisbereich von 1,5-10,9; und
(d) GM-CSF zu IL-2 in einem Verhältnisbereich von 2,2-4,8,
wobei die Hälfte der serumfreien und mitogenfreien Zytokinmischung peritumoral in einem umlaufenden Rand der Tumormasse zu verabreichen ist und die andere Hälfte der Mischung an der submandibulären Lymphkettte ipsilateral zu der Tumormasse dreimal pro Woche über einen Zeitraum von 2 Wochen in einem Bereich von etwa 20 IU bis 1600 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

2. Gebrauch einer therapeutisch aktiven Menge einer serumfreien und mitogenfreien Zytokinmischung zum Herstellen eines Arzneimittels zur Vorsensibilisierung von oralen Plattenepithelkarzinomen vor einer therapeutischen Behandlung,
wobei die Zytokinmischung aus spezifischen Verhältnissen von IL-1β, TNF-α, INF-γ und GM-CSF zu Interleukin 2 (IL-2) wie folgt zusammengesetzt ist:
(a) IL-1β zu IL-2 in einem Verhältnisbereich von 0,4-1,5;
(b) TNF-α zu IL-2 in einem Verhältnisbereich von 3,2-11,3;
(c) IFN-γ zu IL-2 in einem Verhältnisbereich von 1,5-10,9;
(d) GM-CSF zu IL-2 in einem Verhältnisbereich von 2,2-4,8;
wobei die serumfreie und mitogenfreie Zytokinmischung perilymphatisch dreimal pro Woche über einen Zeitraum von 2 Wochen in einem Bereich von etwa 20 IU bis 1600 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

3. Gebrauch nach Anspruch 1 oder 2, wobei die therapeutisch Behandlung aus der Gruppe ausgewählt ist, bestehend aus Chemotherapie, Immuntherapie und Strahlentherapie.

4. Gebrauch nach Anspruch 1, wobei die Hälfte der serumfreien und mitogenfreien Zytokinmischung peritumoral in einem umlaufenden Rand der Tumormasse zu verabreichen ist und die andere Hälfte der Mischung an der submandibulären Lymphkettte ipsilateral zu der Tumormasse dreimal pro Woche über einen Zeitraum von 2 Wochen in einem Bereich von etwa 40 IU bis 800 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

5. Gebrauch nach Anspruch 1, wobei die Hälfte der serumfreien und mitogenfreien Zytokinmischung peritumoral in einem umlaufenden Rand der Tumormasse zu verabreichen ist und die andere Hälfte der Mischung an der submandibulären Lymphkettte ipsilateral zu der Tumormasse dreimal pro Woche über einen Zeitraum von 2 Woche mit 400 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

6. Gebrauch nach Anspruch 1, wobei die Hälfte der serumfreien und mitogenfreien Zytokinmischung peritumoral in einem umlaufenden Rand der Tumormasse zu verabreichen ist und die andere Hälfte der Mischung an der submandibulären Lymphkettte ipsilateral zu der Tumormasse dreimal pro Woche über einen Zeitraum von 2 Wochen mit 800 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

7. Gebrauch nach Anspruch 1, wobei die Hälfte der serumfreien und mitogenfreien Zytokinmischung peritumoral in einem umlaufenden Rand der Tumormasse zu verabreichen ist und die andere Hälfte der Mischung an der submandibulären Lymphkettte ipsilateral zu der Tumormasse fünfmal pro Woche über einen Zeitraum von 2 Wochen mit 800 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

8. Gebrauch nach Anspruch 2, wobei die serumfreie und mitogenfreie Zytokinmischung perilymphatisch dreimal pro Woche über einen Zeitraum von 2 Wochen in einem Bereich von etwa 40 IU bis 800 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

9. Gebrauch nach Anspruch 2, wobei die serumfreie und mitogenfreie Zytokinmischung perilymphatisch dreimal pro Woche über einen Zeitraum von 2 Wochen mit 400 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

10. Gebrauch nach Anspruch 2, wobei die serumfreie und mitogenfreie Zytokinmischung perilymphatisch dreimal pro Woche über einen Zeitraum von 2 Wochen mit 800 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

11. Gebrauch nach Anspruch 2, wobei die serumfreie und mitogenfreie Zytokinmischung perilymphatisch fünfmal pro Woche über einen Zeitraum von 2 Wochen mit 800 IU zu verabreichen ist, wobei IU die internationalen Einheiten für Interleukin-2 repräsentiert, die im 1. Internationalen Standard für humanes IL-2 der Weltgesundheitsorganisation, 86/504, vorgegeben sind.

12. Gebrauch nach Anspruch 1 oder 2, wobei die Zytokinmischung ferner umfasst:
(a) ein Verhältnis von IL-3 zu IL-2 in einem Bereich von 0,38-0,68, vorzugsweise von 0,53 +/- 0,15;
(b) ein Verhältnis von IL-6 zu IL-2 in einem Bereich von 37,2-53,8, vorzugsweise von 46 +/- 5,9;
(c) ein Verhältnis von IL-8 zu IL-2 in einem Bereich von 261-561,5, vorzugsweise von 411 +/- 10,6;
(d) ein Verhältnis IL-1α zu IL-2 in einem Bereich von 0,56-0,94, vorzugsweise von 0,75 +/- 0,19;
(e) ein Verhältnis von IL-10 zu IL-2 in einem Bereich von 2,82-3,22, vorzugsweise von 3,0 +/- 0,18;
(f) ein Verhältnis von IL-16 zu IL-2 in einem Bereich von 1,16-2,84, vorzugsweise von 1,84 +/- 0,68;
(g) ein Verhältnis G-CSF zu IL-2 in einem Bereich von 2,16-3,78, vorzugsweise von 2,97 +/- 0,81;
(h) ein Verhältnis von TNF-β zu IL-2 in einem Bereich von 1,17-2,43, vorzugsweise von 1,8 +/- 0,63;
(i) ein Verhältnis von MIP-1α zu IL-2 in einem Bereich von 15,7-37,16, vorzugsweise von 22,7 +/- 7,0;
(j) ein Verhältnis von MIP-1β zu IL-2 in einem Bereich von 17,1-28,5, vorzugsweise von 22,8 +/- 5,7;
(k) ein Verhältnis von RANTES zu IL-2 in einem Bereich von 2,3-2,7, vorzugsweise von 2,5 +/- 0,13;
(l) ein Verhältnis von EGF zu IL-2 in einem Bereich von 0,267-0,283, vorzugsweise von 0,275 +/- 0,008;
(m) ein Verhältnis von PEG₂ zu IL-2 in einem Bereich von 3,63-5,42, vorzugsweise von 4,5 +/- 0,87;
und/oder
(n) ein Verhältnis von TxB₂ zu IL-2 in einem Bereich von 23,47-25,13, vorzugsweise von 24,3 +/- 0,83.

## Revendications

1. Utilisation d'une quantité thérapeutiquement active d'un mélange de cytokines exempt de sérum et de mitogène pour la fabrication d'un médicament pour la pré-sensibilisation des carcinomes épidermoïdes de la cavité buccale avant un traitement thérapeutique,
dans laquelle ledit mélange de cytokines comprend les rapports spécifiques d'IL-1β, de TNF-α, d'IFN-γ et de GM-CSF à l'interleukine-2 (IL-2) suivants :
(a) IL-1β à IL-2 à une gamme de rapport de 0,4 à 1,5 ;
(b) TNF-α à IL-2 à une gamme de rapport de 3,2 à 11,3;
(c) IFN-γ à IL-2 à une gamme de rapport de 1,5 à 10,9 ; et
(d) GM-CSF à IL-2 à une gamme de rapport de 2,2 à 4,8,
dans laquelle la moitié dudit mélange de cytokines exempt de sérum et de mitogène doit être administrée par voie péritumorale au niveau d'un bord circonférentiel de la masse tumorale et l'autre moitié dudit mélange doit être administrée au niveau de la chaîne ganglionnaire sous-maxillaire ipsilatérale à la masse tumorale trois fois par semaine sur une période de deux semaines dans une gamme d'environ 20 UI à 1 600 UI, où UI représente les unités internationales pour l'interleukine-2 indiquées dans la première norme internationale de l'Organisation mondiale de la Santé pour l'IL-2 humaine, 86/504.

2. Utilisation d'une quantité thérapeutique active d'un mélange de cytokines exempt de sérum et de mitogène pour la fabrication d'un médicament pour la pré-sensibilisation des carcinomes épidermoïdes de la cavité buccale avant un traitement thérapeutique,
dans laquelle ledit mélange de cytokines comprend les rapports spécifiques d'IL-1β, de TNF-α, d'IFN-γ et de GM-CSF à l'interleukine-2 (IL-2) suivants:
(a) IL-1β à IL-2 à une gamme de rapport de 0,4 à 1,5 ;
(b) TNF-α à IL-2 à une gamme de rapport de 3,2 à 11,3;
(c) IFN-γ à IL-2 à une gamme de rapport de 1,5 à 10,9 ; et
(d) GM-CSF à IL-2 à une gamme de rapport de 2,2 à 4,8,
dans laquelle ledit mélange de cytokines exempt de sérum et de mitogène doit être administré par voie périlymphatique trois fois par semaine sur une période de deux semaines dans une gamme d'environ 20 UI à 1 600 UI, où UI représente les unités internationales pour l'interleukine-2 indiquées dans la première norme internationale de l'Organisation mondiale de la Santé pour l'IL-2 humaine, 86/504.

3. Utilisation selon la revendication 1 ou 2, dans laquelle ledit traitement thérapeutique est choisi dans le groupe constitué par la chimiothérapie, l'immunothérapie et la radiothérapie.

4. Utilisation selon la revendication 1, dans laquelle la moitié dudit mélange de cytokines exempt de sérum et de mitogène doit être administrée par voie péritumorale au niveau d'un bord circonférentiel de la masse tumorale et l'autre moitié dudit mélange doit être administrée au niveau de la chaîne ganglionnaire sous-maxillaire ipsilatérale à la masse tumorale trois fois par semaine sur une période de deux semaines dans une gamme d'environ 40 UI à 800 UI, où UI représente les unités internationales pour l'interleukine-2 indiquées dans la première norme internationale de l'Organisation mondiale de la Santé pour l'IL-2 humaine, 86/504.

5. Utilisation selon la revendication 1, dans laquelle la moitié dudit mélange de cytokines exempt de sérum et de mitogène doit être administrée par voie péritumorale au niveau d'un bord circonférentiel de la masse tumorale et l'autre moitié dudit mélange doit être administrée au niveau de la chaîne ganglionnaire sous-maxillaire ipsilatérale à la masse tumorale trois fois par semaine sur une période de deux semaines à 400 UI, où UI représente les unités internationales pour l'interleukine-2 indiquées dans la première norme internationale de l'Organisation mondiale de la Santé pour l'IL-2 humaine, 86/504.

6. Utilisation selon la revendication 1, dans laquelle la moitié dudit mélange de cytokines exempt de sérum et de mitogène doit être administrée par voie péritumorale au niveau d'un bord circonférentiel de la masse tumorale et l'autre moitié dudit mélange doit être administrée au niveau de la chaîne ganglionnaire sous-maxillaire ipsilatérale à la masse tumorale trois fois par semaine sur une période de deux semaines à 800 UI, où UI représente les unités internationales pour l'interleukine-2 indiquées dans la première norme internationale de l'Organisation mondiale de la Santé pour l'IL-2 humaine, 86/504.

7. Utilisation selon la revendication 1, dans laquelle la moitié dudit mélange de cytokines exempt de sérum et de mitogène doit être administrée par voie péritumorale au niveau d'un bord circonférentiel de la masse tumorale et l'autre moitié dudit mélange doit être administrée au niveau de la chaîne ganglionnaire sous-maxillaire ipsilatérale à la masse tumorale cinq fois par semaine sur une période de deux semaines à 800 UI, où UI représente les unités internationales pour l'interleukine-2 indiquées dans la première norme internationale de l'Organisation mondiale de la Santé pour l'IL-2 humaine, 86/504.

8. Utilisation selon la revendication 2, dans laquelle ledit mélange de cytokines exempt de sérum et de mitogène doit être administré par voie périlymphatique trois fois par semaine sur une période de deux semaines dans une gamme d'environ 40 UI à 800 UI, où UI représente les unités internationales pour l'interleukine-2 indiquées dans la première norme internationale de l'Organisation mondiale de la Santé pour l'IL-2 humaine, 86/504.

9. Utilisation selon la revendication 2, dans laquelle ledit mélange de cytokines exempt de sérum et de mitogène doit être administré par voie périlymphatique trois fois par semaine sur une période de deux semaines à 400 UI, où UI représente les unités internationales pour l'interleukine-2 indiquées dans la première norme internationale de l'Organisation mondiale de la Santé pour l'IL-2 humaine, 86/504.

10. Utilisation selon la revendication 2, dans laquelle ledit mélange de cytokines exempt de sérum et de mitogène doit être administré par voie périlymphatique trois fois par semaine sur une période de deux semaines à 800 UI, où UI représente les unités internationales pour l'interleukine-2 indiquées dans la première norme internationale de l'Organisation mondiale de la Santé pour l'IL-2 humaine, 86/504.

11. Utilisation selon la revendication 2, dans laquelle ledit mélange de cytokines exempt de sérum et de mitogène doit être administré par voie périlymphatique cinq fois par semaine sur une période de deux semaines à 800 UI, où UI représente les unités internationales pour l'interleukine-2 indiquées dans la première norme internationale de l'Organisation mondiale de la Santé pour l'IL-2 humaine, 86/504.

12. Utilisation selon la revendication 1 ou 2, dans laquelle le mélange de cytokines comprend en outre :
(a) un rapport d'IL-3 à IL-2 dans une gamme comprise entre 0,38 et 0,68, de préférence à 0,53 +/- 0,15 ;
(b) un rapport d'IL-6 à IL-2 dans une gamme comprise entre 37,2 et 53,8, de préférence à 46 +/- 5,9 ;
(c) un rapport d'IL-8 à IL-2 dans une gamme comprise entre 261 et 561,5, de préférence à 411 +/- 10,6;
(d) un rapport d'IL-1α à IL-2 dans une gamme comprise entre 0,56 et 0,94, de préférence à 0,75 +/- 0,19 ;
(e) un rapport d'IL-10 à IL-2 dans une gamme comprise entre 2,82 et 3,22, de préférence à 3,0 +/- 0,18 ;
(f) un rapport d'IL-16 à IL-2 dans une gamme comprise entre 1,16 et 2,84, de préférence à 1,84 +/- 0,68 ;
(g) un rapport de G-CSF à IL-2 dans une gamme comprise entre 2,16 et 3,78, de préférence à 2,97 +/- 0,81 ;
(h) un rapport de TNF-β à IL-2 dans une gamme comprise entre 1,17 et 2,43, de préférence à 1,8 +/- 0,63 ;
(i) un rapport de MIP-1α à IL-2 dans une gamme comprise entre 15,7 et 37,16, de préférence à 22,7 +/- 7,0 ;
(j) un rapport de MIP-1β à IL-2 dans une gamme comprise entre 17,1 et 28,5, de préférence à 22,8 +/- 5,7 ;
(k) un rapport de RANTES à IL-2 dans une gamme comprise entre 2,3 et 2,7, de préférence à 2,5 +/- 0,13 ;
(l) un rapport d'EGF à IL-2 dans une gamme comprise entre 0,267 et 0,283, de préférence à 0,275 +/- 0,008 ;
(m) un rapport de PGE₂ à IL-2 dans une gamme comprise entre 3,63 et 5,42, de préférence à 4,5 +/- 0,87 ; et/ou
(n) un rapport de TxB₂ à IL-2 dans une gamme comprise entre 23,47 et 25,13, de préférence à 24,3 +/- 0,83.
